(19) Europäisches Patentamt
European Patent Office
Office européen des brevets

(11) **EP 3 697 751 B1**

(12) **EUROPÄISCHE PATENTSCHRIFT**

(45) Veröffentlichungstag und Bekanntmachung des Hinweises auf die Patenterteilung:
**06.12.2023 Patentblatt 2023/49**

(21) Anmeldenummer: **18792915.3**

(22) Anmeldetag: **19.10.2018**

(51) Internationale Patentklassifikation (IPC):
***C07C 41/56*** (2006.01) ***C07C 41/50*** (2006.01)
***C07C 43/30*** (2006.01) ***B01J 23/881*** (2006.01)
***C07C 45/38*** (2006.01) ***C07C 47/04*** (2006.01)

(52) Gemeinsame Patentklassifikation (CPC):
(C-Sets verfügbar)
**C07C 41/56; B01J 23/881; C07C 41/50; C07C 45/38** (Forts.)

(86) Internationale Anmeldenummer:
**PCT/EP2018/078788**

(87) Internationale Veröffentlichungsnummer:
**WO 2019/077140 (25.04.2019 Gazette 2019/17)**

(54) **VERFAHREN ZUR HERSTELLUNG VON POLYOXYMETHYLENDIMETHYLETHER**

METHOD FOR PRODUCING POLYOXYMETHYLENE DIMETHYL ETHER

PROCÉDÉ DE PRÉPARATION DE DIMÉTHYLÉTHER DE POLYOXYMÉTHYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.10.2017 DE 102017218782**

(43) Veröffentlichungstag der Anmeldung:
**26.08.2020 Patentblatt 2020/35**

(73) Patentinhaber: **Fraunhofer-Gesellschaft zur Förderung der angewandten Forschung e.V.
80686 München (DE)**

(72) Erfinder:
- **M. SC. OUDA, Mohamed
79115 Freiburg (DE)**
- **WHITE, Robin
5211 SX ’s-Hertogenbosch (NL)**
- **SCHAADT, Achim
79111 Freiburg (DE)**

(74) Vertreter: **Maiwald GmbH
Elisenhof
Elisenstraße 3
80335 München (DE)**

(56) Entgegenhaltungen:
**EP-A1- 2 228 359**
**WO-A1-2007/034264**
**DE-A1-102004 053 839**
**US-A1- 2015 291 722**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 697 751 B1

(52) Gemeinsame Patentklassifikation (CPC): (Forts.)

C-Sets
**C07C 41/50, C07C 43/30;**
**C07C 41/56, C07C 43/30;**
**C07C 45/38, C07C 47/04**

**Beschreibung**

Die vorliegende Erfindung betrifft Verfahren zur Herstellung von Polyoxymethylendimethylether (OME) und eine Verwendung eines Katalysators in einem Verfahren zur Herstellung von Polyoxymethylendimethylether (OME).

Synthetische Energieträger, welche nicht auf Basis von Rohöl oder Erdgas hergestellt werden, können die Abhängigkeit von fossilen Energiequellen, sowie die aus deren Verwendung entstehende Umweltbelastung verringern. Ein Beispiel für einen solchen Energieträger sind Polyoxymethylendimethylether (OMEs). Polyoxymethylendimethylether (OMEs) können aus Kohlenstoffdioxid und Wasser hergestellt werden und weisen, soweit regenerative Energieträger für deren Herstellung eingesetzt werden, bei der Umsetzung als Kraftstoff einen geschlossenen Kohlenstoffdioxidkreislauf auf, der das Kriterium der Kohlenstoffdioxid-Neutralität erfüllt.

Darüber hinaus bietet die Nutzung der Polyoxymethylendimethylether (OMEs) als Energieträger weitere Vorteile. Polyoxymethylendimethylether (OMEs) weisen keine Kohlenstoff-Kohlenstoff-Bindungen auf und besitzt außerdem einen hohen Anteil an Sauerstoff. Polyoxymethylendimethylether (OMEs) verbrennen deshalb rußfrei und schonen damit sowohl den Verbrennungsmotor und nachgeschaltete Filterelemente als auch die Umwelt. Polyoxymethylendimethylether mit drei bis fünf repetitiven Einheiten (OME 3-5) sind dabei von besonderem Interesse.

Derzeit sind insbesondere zwei Herstellungsverfahren für Polyoxymethylendimethylether mit drei bis fünf repetitiven Einheiten (OME 3-5) im Einsatz, eine wässrige Syntheseroute und eine wasserfreie Syntheseroute.

Die EP 2 228 359 A1 beschreibt ein Verfahren zur Herstellung von Polyoxymethylendimethylether aus Methanol, umfassend die Kontaktreaktion von Methanol mit Sauerstoff in der Gegenwart eines Katalysators, wobei der Katalysator ein Eisen enthaltendes Metallmischoxid und einen Molekularsieb mit einer sauren katalytischen Aktivität enthält.

Bei der wässrigen Syntheseroute wird Methanol mit einer Formaldehydquelle wie Formalin oder para-Formaldehyd umgesetzt.

Die Isolierung von Polyoxymethylendimethylether mit drei bis fünf repetitiven Einheiten (OME 3-5) bringt dabei eine aufwendige und umständliche Aufarbeitung mit sich, insbesondere da weitere Nebenprodukte gebildet werden, beispielsweise Wasser durch eine Kondensation zwischen Methanol und Formaldehyd und Methylenglykol sowie Hemiformal durch eine entsprechende Acetalisierung. Bei der wasserfreien Syntheseroute werden "Methyl-Capping-Gruppen" wie Dimethoxymethan (OME 1) und/oder Dimethylether (DME) mit einer wasserfreien Formaldehydquelle wie Trioxan (TRI) oder para-Formaldehyd (p-FA) umgesetzt. Die für diese Syntheseroute benötigten Edukte werden typischerweise getrennt voneinander über eigenständige Syntheserouten hergestellt, was zu einer umständlichen und/oder energieintensiven Reaktionsführung führt.

Vor diesem Hintergrund war die Herstellung von Polyoxymethylendimethylether mit drei bis fünf repetitiven Einheiten (OME 3-5) für den Einsatz als Kraftstoffadditiv großtechnisch nicht interessant. Es besteht ein Bedarf die bekannten Verfahren zur Herstellung von Polyoxymethylendimethylether mit drei bis fünf repetitiven Einheiten (OME 3-5) zu verbessern, insbesondere zu vereinfachen und effizienter zu gestalten, so dass eine großtechnische Anwendung möglich wird.

Die Aufgabe der vorliegenden Erfindung ist es, die Nachteile der bekannten Verfahren zur Herstellung von Polyoxymethylendimethylether mit drei bis fünf repetitiven Einheiten (OME 3-5) zu beseitigen.

Diese und weitere Aufgaben werden mit der vorliegenden Erfindung gelöst. Die Erfindung ist in den unabhängigen Patentansprüchen angegeben, vorteilhafte Ausführungsformen werden in den abhängigen Patentansprüchen beschrieben.

**Kurzbeschreibung der Erfindung:**

Die vorliegende Erfindung stellt ein Verfahren zur Herstellung von Polyoxymethylendimethylether (OME) gemäß Anspruch 1, ein Verfahren zur Herstellung von Polyoxymethylendimethylether (OME) gemäß Anspruch 3 und eine Verwendung eines Katalysators in einem Verfahren zur Herstellung von Polyoxymethylendimethylether (OME) gemäß Anspruch 15 bereit. Bevorzugte Ausführungsformen der Erfindung sind in den abhängigen Ansprüchen beschrieben.

In einem ersten Aspekt betrifft die vorliegende Erfindung ein Verfahren zur Herstellung von Polyoxymethylendimethylether (OME), umfassend einen ersten Verfahrensschritt, bei dem aus einem Feed (FD), enthaltend Methanol (ME), in einem ersten Reaktor (R-1) in Gegenwart eines ersten Katalysators (K-1) ein Reaktionsgemisch (RG-1) gebildet wird, wobei das Reaktionsgemisch (RG-1) Dimethoxymethan (OME 1) und Formaldehyd (FA) umfasst.

Das Reaktionsgemisch (RG-1) enthält Dimethoxymethan (OME 1) und Formaldehyd (FA) in einem Stoffmengenverhältnis (mol/mol) Dimethoxymethan (OME 1) zu Formaldehyd (FA) in einem Bereich von 0,1 bis 2,0, bevorzugt in einem Bereich von 0,3 bis 1,5.

In einem zweiten Verfahrensschritt wird das Reaktionsgemisch (RG-1) einem zweiten Reaktor (R-2) zugeführt und in Gegenwart eines zweiten Katalysators (K-2) zu Polyoxymethylendimethylether (OME) umgesetzt.

In einem zweiten Aspekt betrifft die vorliegende Erfindung ein Verfahren zur Herstellung von Polyoxymethylendimethylether (OME) umfassend einen Verfahrensschritt, bei dem aus einem Feed (FD), enthaltend Methanol (ME),

in einem Reaktor (R-1) in Gegenwart eines Katalysators (K-1) ein Reaktionsgemisch (RG-1) gebildet wird, wobei das Reaktionsgemisch (RG-1) Dimethoxymethan (OME 1) und Formaldehyd (FA), vorzugsweise in einem Stoffmengenverhältnis (mol/mol) Dimethoxymethan (OME 1) zu Formaldehyd (FA) in einem Bereich von 0,3 bis 1,5, umfasst und nicht umgesetztes Methanol (ME) und neben Dimethoxymethan (OME 1) und Formaldehyd (FA) gebildetes Wasser aus dem Reaktionsgemisch (RG-1) entfernt werden.

Hierbei kann das Reaktionsgemisch (RG-1) ebenfalls in einem zweiten Verfahrensschritt einem zweiten Reaktor (R-2) zugeführt und in Gegenwart eines zweiten Katalysators (K-2) zu Polyoxymethylendimethylether (OME) umgesetzt werden.

Gemäß einer Ausführungsform werden nicht umgesetztes Methanol (ME) und neben Dimethoxymethan (OME 1) und Formaldehyd (FA) gebildetes Wasser über Destillation und/oder einen Adsorptionsprozess und/oder ein Absorptionsverfahren und/oder eine Membran aus dem Reaktionsgemisch (RG-1) entfernt.

Gemäß einer Ausführungsform werden nicht umgesetztes Methanol (ME) und neben Dimethoxymethan (OME 1) und Formaldehyd (FA) gebildetes Wasser über eine Membran und optional über eine Destillation und/oder einen Adsorptionsprozess und/oder über ein Absorptionsverfahren aus dem Reaktionsgemisch (RG-1) entfernt.

Besonders bevorzugt handelt es sich bei der Destillation um eine fraktionierte Destillation.

Das Verfahren kann die folgenden Verfahrensschritte umfassen:

(a) Bereitstellen eines Feed (FD), umfassend Methanol (ME), und überführen in den ersten Reaktor (R-1);

(b) Reaktion von dem in dem Feed (FD) enthaltenem Methanol (ME) in Gegenwart des ersten Katalysators (K-1) unter Bildung eines ersten Reaktionsgemisches (RG-1), wobei das erste Reaktionsgemisch (RG-1) Dimethoxymethan (OME 1) und Formaldehyd (FA) umfasst;

(c) Aufarbeiten des in dem ersten Reaktor (R-1) gebildeten Reaktionsgemisches (RG-1) durch das Abtrennen von Wasser und Methanol über eine Destillation und/oder über einen Adsorptionsprozess und/oder über ein Absorptionsverfahren, so dass ein Reaktionsgemisch (RG-1A) erhalten wird; oder

(d) Aufarbeiten des in dem ersten Reaktor (R-1) gebildeten Reaktionsgemisches (RG-1) durch das Abtrennen von Wasser und Methanol über eine Membran und optional über eine Destillation und/oder einen Adsorptionsprozess und/oder über ein Absorptionsverfahren, so dass ein Reaktionsgemisch (RG-1B) erhalten wird,

(e) Überführen des Reaktionsgemisches (RG-1A) oder des Reaktionsgemisches (RG-1B) in den zweiten Reaktor (R-2);

(f) Reaktion des Reaktionsgemisches (RG-1A) oder des Reaktionsgemisches (RG-1B) in Gegenwart des zweiten Katalysators (K-2) unter Bildung eines Reaktionsgemisches (RG-2), das Reaktionsgemisch (RG-2) umfassend Polyoxymethylendimethylether mit ein bis 25 repetitiven Einheiten (OME 1-25) gemäß Formel (I):

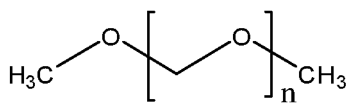 (I)

wobei n = 1-25;

(g) Aufarbeitung des in dem zweiten Reaktor (R-2) gebildeten Reaktionsgemisches (RG-2) durch Abtrennen von Polyoxymethylendimethylether mit drei bis fünf repetitiven Einheiten (OME 3-5) gemäß Formel (II):

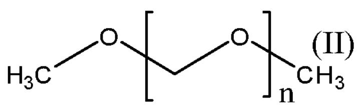 (II)

wobei n = 3-5.

Besonders bevorzugt handelt es sich bei der Destillation in Schritt (c) um eine fraktionierte Destillation.

Der Feed (FD) kann Methanol (ME) in einer Menge in einem Bereich von 30 bis 90 Vol.-% umfassen, bezogen auf das Gesamtvolumen des Feed (FD).

Die Reaktion von Methanol (ME) in Gegenwart des Katalysators (K-1) kann bei einer Temperatur in einem Bereich von 150 bis 400 °C, einem Druck in einem Bereich von 0,5 bis 1,5 bar und einer Raumgeschwindigkeit (GHSV) in einem Bereich von 3000 bis 35000 $h^{-1}$ durchgeführt werden.

Der in dem ersten Reaktor (R-1) eingesetzte Katalysator (K-1) kann Eisen und Molybdän als aktive Katalysatorkomponenten enthalten, wobei Eisen und Molybdän vorzugsweise als Oxide, Mischoxide oder deren Mischungen vorliegen, und wobei das atomare Stoffmengenverhältnis (mol/mol) von Molybdän zu Eisen in dem Katalysator (K-1) vorzugsweise in einem Bereich von 4.0 bis 2,0 liegt.

Nicht umgesetztes Methanol (ME) und neben Dimethoxymethan (OME 1) und Formaldehyd (FA) gebildetes Wasser können aus dem Reaktionsgemisch (RG-1) über eine Behandlung mit einer Membran, vorzugsweise einer Polymermembran mit polaren Gruppen entfernt werden, insbesondere über eine Behandlung mit einer Polymermembran umfassend ein Copolymer aus Tetrafluorethylen und Perfluor-3,6-dioxa-4-methyl-7-octen-sulfonsäure, oder eine Membran, die Zeolithe umfasst.

Die Stoffmenge der in dem ersten Reaktor (R-1) neben Dimethoxymethan (OME 1) und Formaldehyd (FA) gebildeten organischen Bestandteile kann weniger als 10 mol% betragen, bezogen auf die gesamte Stoffmenge der gebildeten organischen Bestandteile in dem Reaktionsgemisch (RG-1).

Wenigstens ein Teil des bei der Aufarbeitung des Reaktionsgemisches (RG-2) isolierten Dimethoxymethans (OME 1) und/oder Polyoxymethylendimethylethers mit zwei repetitiven Einheiten (OME 2) kann mit dem aus dem ersten Reaktor (R-1) erhaltenen Reaktionsgemisch (RG-1) vereint werden.

In einem dritten Aspekt betrifft die vorliegende Erfindung die Verwendung eines Katalysators (K-1) in einem Verfahren zur Herstellung von Polyoxymethylendimethylether (OME) gemäß dem ersten Aspekt oder gemäß dem zweiten Aspekt zur Steuerung des aus Methanol (ME) gebildeten Stoffmengenverhältnisses von Dimethoxymethan (OME 1) und Formaldehyd (FA).

## Detaillierte Beschreibung der Erfindung:

Es wird ein Verfahren zur Herstellung von Polyoxymethylendimethylether (OME) beschrieben, insbesondere ein Verfahren zur Herstellung von Polyoxymethylendimethylether mit drei bis fünf repetitiven Einheiten (OME 3-5), i.e. Polyoxymethylendimethylether gemäß Formel (I):

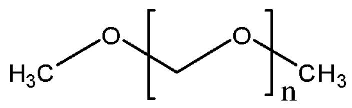

(I)

wobei n = 3-5.

Das Verfahren zur Herstellung von Polyoxymethylendimethylether (OME), insbesondere das Verfahren zur Herstellung von Polyoxymethylendimethylether mit drei bis fünf repetitiven Einheiten (OME 3-5), ausgehend von Methanol (ME), umfasst vorzugsweise nicht mehr als zwei Reaktionsstufen.

In anderen Worten, es handelt sich um ein Verfahren zur Herstellung von Polyoxymethylendimethylether (OME), insbesondere um ein Verfahren zur Herstellung von Polyoxymethylendimethylether mit drei bis fünf repetitiven Einheiten (OME 3-5), bei dem Methanol (ME), welches als einziges organisches Edukt von außerhalb zugeführt wird, in zwei Reaktionsstufen zu Polyoxymethylendimethylether (OME), insbesondere zu Polyoxymethylendimethylether mit drei bis fünf repetitiven Einheiten (OME 3-5), umgesetzt wird.

Die bekannten Verfahren zur Herstellung von Polyoxymethylendimethylether (OME) verwenden ebenfalls Methanol. Dieses wird jedoch nicht als Edukt zur Synthese der Polyoxymethylendimethylether (OME) eingesetzt, sondern in eigenständigen, von der eigentlichen Herstellung der Polyoxymethylendimethylether (OME) völlig unabhängigen Reaktionen zur Herstellung der benötigten Edukte, i.e. Formaldehyd (FA), Trioxan (TRI), Dimethoxymethan (OME 1), Dimethylether (DME) und/oder para-Formaldehyd (p-FA). Diese werden getrennt voneinander hergestellt und aufgearbeitet. Die so hergestellten Verbindungen werden anschließend in getrennten Feeds von außen zugeführt und zu Polyoxymethylendimethylethern (OME) umgesetzt.

Im Gegensatz hierzu wird in dem hier beschriebenen Verfahren Methanol (ME) vorzugsweise als einziges organisches Edukt von außerhalb zugeführt, vorzugsweise über einen Feed (FD) der ein gasförmiges Gemisch aus Methanol (ME) und Sauerstoff umfasst, insbesondere über einen Feed (FD) der ein gasförmiges Gemisch aus Methanol (ME) und Luftsauerstoff umfasst.

Die Erfindung betrifft demzufolge in einem ersten Aspekt ein Verfahren zur Herstellung von Polyoxymethylendimethylether (OME), umfassend einen Verfahrensschritt bei dem aus einem Feed (FD) enthaltend Methanol (ME) in einem Reaktor (R-1) in Gegenwart eines Katalysators (K-1) ein Reaktionsgemisch (RG-1) gebildet wird, wobei das Reaktionsgemisch (RG-1) Dimethoxymethan (OME 1) und Formaldehyd (FA) enthält.

Das in dem Verfahren zur Herstellung von Polyoxymethylendimethylether (OME) gebildete Reaktionsgemisch (RG-1) enthält Dimethoxymethan (OME 1) und Formaldehyd (FA) in einem Stoffmengenverhältnis (mol/mol) von Dimethoxymethan (OME 1) zu Formaldehyd (FA) in einem Bereich von 0,1 bis 2,0, vorzugsweise in einem Bereich von 0,3 bis 1,5, weiter bevorzugt in einem Bereich von 0,3 bis 0,8, noch weiter bevorzugt in einem Bereich von 0,3 bis 0,5, darüber hinaus bevorzugt in einem Bereich von 0,5 bis 1,3, noch weiter bevorzugt in einem Bereich von 0,8 bis 1,0, ferner bevorzugt in einem Bereich von 0,2 bis 1,5, weiter bevorzugt in einem Bereich von 0,5 bis 1,2.

Alternativoder zusätzlich kann nicht umgesetztes Methanol (ME) und neben Dimethoxymethan (OME 1) und

Formaldehyd (FA) gebildetes Wasser aus dem Reaktionsgemisch (RG-1) entfernt werden.

Der Feed (FD) kann Methanol (ME), insbesondere in Form eines Gemisches aus Methanol (ME) und Luft, in einer Menge von mindestens 30 Vol.-% enthalten, vorzugsweise mindestens 35 Vol.-%, weiter bevorzugt mindestens 40 Vol.%, beispielsweise in einer Menge in einem Bereich von 30 bis 90 Vol.-%, vorzugsweise in einem Bereich 35 bis 75 Vol.-%, weiter bevorzugt in einem Bereich von 40 bis 60 Vol.-%, bezogen auf das Gesamtvolumen des Feed (FD).

Die Umsatzrate von Methanol ($UR_{ME}$) in dem Reaktor (R-1) kann mindestens 30 mol% betragen, vorzugsweise mindestens 40 mol%, weiter bevorzugt mindestens 50 mol%, bezogen auf die gesamte Stoffmenge des Methanols (ME) dass über den Feed (FD) in den Reaktor (R-1) eingebracht wurde.

Die Umsatzrate von Methanol ME zu Dimethoxymethan OME 1 und Formaldehyd FA ($UR_{OME1FA}$) in dem ersten Reaktor (R-1), also die Selektivität der Reaktion von Methanol (ME) bezogen auf die Reaktionsprodukte Dimethoxymethan (OME 1) und Formaldehyd (FA) kann mindestens 50 mol% betragen, vorzugsweise mindestens 70 mol%, weiter bevorzugt mindestens 85 mol%, bezogen auf die gesamte Stoffmenge des über den Feed (FD) in den Reaktor (R-1) eingebrachten Methanols (ME)

Die Bestimmung der Umsatzraten $UR_{ME}$ und $UR_{OME1FA}$ werden weiter unten genauer beschrieben.

Das Verfahren zur Herstellung von Polyoxymethylendimethylether (OME), insbesondere zur Herstellung von Polyoxymethylendimethylether mit drei bis fünf repetitiven Einheiten (OME 3-5), umfasst vorzugsweise die folgenden Verfahrensschritte:

(a) Bereitstellen eines Feed (FD) umfassend Methanol (ME) und überführen in einen ersten Reaktor (R-1);

(b) Reaktion von dem in dem Feed (FD) enthaltenem Methanol (ME) in Gegenwart eines ersten Katalysators (K-1) unter Bildung eines ersten Reaktionsgemisches (RG-1), wobei das erste Reaktionsgemisch (RG-1) Dimethoxymethan (OME 1) und Formaldehyd (FA) umfasst;

(c) Aufarbeiten des in dem ersten Reaktor (R-1) gebildeten Reaktionsgemisches (RG-1) durch das Abtrennen von Wasser und Methanol über eine Destillation und/oder über einen Adsorptionsprozess und/oder über ein Absorptionsverfahren, so dass ein Reaktionsgemisch (RG-1A) erhalten wird; oder

(d) Aufarbeiten des in dem ersten Reaktor (R-1) gebildeten Reaktionsgemisches (RG-1) durch das Abtrennen von Wasser und Methanol über eine Membran und optional über eine Destillation und/oder über einen Adsorptionsprozess und/oder über ein Absorptionsverfahren, so dass ein Reaktionsgemisch (RG-1B) erhalten wird;

(e) Überführen des Reaktionsgemisches (RG-1A) oder des Reaktionsgemisches (RG-1B) in einem zweiten Reaktor (R-2);

(f) Reaktion des Reaktionsgemisches (RG-1A) oder des Reaktionsgemisches (RG-1B) in Gegenwart eines zweiten Katalysators (K-2) unter Bildung eines Reaktionsgemisches (RG-2), das Reaktionsgemisch (RG-2) umfassend Polyoxymethylendimethylether mit ein bis 25 repetitiven Einheiten (OME 1-25) gemäß Formel (I):

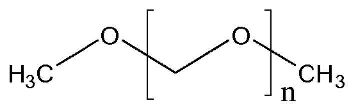 (I)

wobei n = 1-25;

(g) Aufarbeitung des in dem zweiten Reaktor (R-2) gebildeten Reaktionsgemisches (RG-2) durch Abtrennen von Polyoxymethylendimethylether mit drei bis fünf repetitiven Einheiten (OME 3-5) gemäß Formel (II):

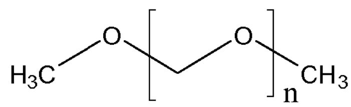 (II)

wobei n = 3-5.

Besonders bevorzugt handelt es sich bei der Destillation in Schritt (c) um eine fraktionierte Destillation.

In einem ersten Verfahrensschritt wird das Methanol (ME) über den Feed (FD) in einen ersten Reaktor (R-1) überführt, in welchem das Methanol (ME) in Gegenwart des Katalysators (K-1) umgesetzt wird.

Ziel dieser Umsetzung ist es, in einem einzigen Verfahrensschritt alle für die Herstellung der Polyoxymethylendimethylether (OME), insbesondere alle für die Herstellung der Polyoxymethylendimethylether mit drei bis fünf repetitiven Einheiten (OME 3-5), benötigten organischen Edukte herzustellen, so dass diese anschließend in Form eines entsprechenden Reaktionsgemisches, ohne vorherige Isolierung, zur Herstellung der Polyoxymethylendimethylether (OME), insbesondere zur Herstellung der Polyoxymethylendimethylether mit drei bis fünf repetitiven Einheiten (OME

3-5), eingesetzt werden können.

Für die Herstellung der Polyoxymethylendimethylether (OMEs), insbesondere für die Herstellung der Polyoxymethylendimethylether mit drei bis fünf repetitiven Einheiten (OME 3-5), werden Dimethoxymethan (OME 1) und Formaldehyd (FA) benötigt.

Die Synthese von Dimethoxymethan (OME 1) und Formaldehyd (FA) aus Methanol (ME) in einem einzigen Verfahrensschritt ist ein wesentlicher Bestandteil des beschriebenen Verfahrens und trägt ganz entscheidend zur Effizienz und Wirtschaftlichkeit bei.

Es ist demzufolge erforderlich, dass das über Feed (FD) in den ersten Reaktor (R-1) eingetragene Methanol (ME) sowohl zu Dimethoxymethan (OME 1) als auch zu Formaldehyd (FA) umgesetzt wird.

Die direkte Synthese von Dimethoxymethan (OME 1) aus Methanol wurde in der einschlägigen Literatur bislang nur wenig diskutiert. Die in diesem Zusammenhang eingesetzten Katalysatoren zeigen nur eine geringe Selektivität gegenüber Dimethoxymethan (OME 1), so dass typischerweise größere Mengen an Nebenprodukt gebildet werden. Außerdem ist es für das beschrieben Verfahren erforderlich, dass ein gewisser Anteil des *in situ* gebildeten Formaldehyds (FA) nicht zu Dimethoxymethan (OME 1) hydrolysiert, so dass ein Gemisch aus Formaldehyds (FA) und Dimethoxymethan (OME 1) erhalten wird. Dieses Gemisch aus Formaldehyd (FA) und Dimethoxymethan (OME 1) sollte außerdem möglichst frei von Nebenprodukten sein.

Der eingesetzte Katalysator (K-1) ist deshalb vorzugsweise ein bifunktionaler Katalysator, welcher einerseits funktionelle Gruppen mit Oxidationswirkung und andererseits funktionelle Gruppen mit Säurewirkung aufweist. Das Verhältnis der in dem ersten Reaktor (R-1) gebildeten Produkte, insbesondere das Verhältnis von Dimethoxymethan (OME 1) zu Formaldehyd (FA), kann über das Verhältnis der funktionellen Gruppen, insbesondere über das Verhältnis der funktionellen Gruppen mit Oxidationswirkung zu den funktionellen Gruppen mit Säurewirkung eingestellt werden.

Es kann ein Katalysator eingesetzt werden, der Eisen und/oder Molybdän als aktive Katalysatorkomponente(n) enthält, insbesondere kann ein Katalysator eingesetzt werden, der Eisen und/oder Molybdän in Form von Oxiden, Mischoxiden und/oder Heteropolyverbindungen enthält, insbesondere in Form von Oxiden und/oder Mischoxiden. Der Katalysator kann außerdem Eisen und/oder Molybdän in Form von Halogeniden, insbesondere in Form von Chloriden, enthalten.

Geeignete Vertreter solcher aktiven Katalysatorkomponenten sind beispielsweise $Fe_2O_3$, MoOs und/oder $Fe_2(MoO_4)_3$.

In einer Ausführungsform umfasst der Katalysator (K-1) Eisen und Molybdän in Form von Oxiden und/oder Mischoxiden als aktive Katalysatorkomponenten, insbesondere in Form von MoOs und $Fe_2(MoO_4)_3$.

Entsprechende Katalysatoren werden beispielsweise in J. Gomay, X. Sécordel, G. Tesquet, B. de Ménorval, S. Cristol, P. Fongarland, M. Capron, L. Duhamel, E. Payen, J.-L. Dubois und F. Dumeignil, Green Chem., 2010, 12(10), 1722, J. M. Tatibouët, Applied Catalysis A: General, 1997, 148(2), 213 und K.-a. Thavornprasert, M. Capron, L. Jalowiecki-Duhamel and F. Dumeignil, Catal. Sci. Technol., 2016, 6(4), 958 beschrieben.

In einer Ausführungsform bestehen die aktiven Katalysatorkomponenten des Katalysators (K-1) aus Eisen und Molybdän in Form von Oxiden, Mischoxiden und/oder Heteropolyverbindungen, sowie optional Eisen und/oder Molybdän in Form von Halogeniden, insbesondere können die aktiven Katalysatorkomponenten des Katalysators (K-1) aus Eisen und Molybdän in Form von Oxiden und Mischoxiden, wie MoOs und $Fe_2(MoO_4)_3$, und optional Eisen und/oder Molybdän in Form von Chloriden, wie $Mo_xCl_y$ oder $Fe_xCl_y$, bestehen.

In einer bevorzugten Ausführungsform bestehen die aktiven Katalysatorkomponenten des Katalysators (K-1) aus Eisen und Molybdän in Form von Oxiden und Mischoxiden, sowie optional Eisen und Molybdän in Form von Chloriden.

Für den Fall dass der Katalysator (K-1) Eisen und Molybdän als aktive Katalysatorkomponenten enthält, wird das atomare Stoffmengenverhältnis von Molybdän zu Eisen aus den entsprechenden Oxiden, Mischoxiden, Heteropolyverbindungen und Halogenen vorzugsweise so eingestellt, dass es in einem Bereich von 4,0 bis 2,0 liegt, vorzugsweise in einem Bereich von 3,5 bis 2,5, weiter bevorzugt in einem Bereich von 3,2 bis 2,8.

In einer Ausführungsform umfasst der Katalysator (K-1) neben Eisen und/oder Molybdän keine anderen aktive(n) Katalysatorkomponente(n), insbesondere umfasst der Katalysator (K-1) neben Eisen und/oder Molybdän in Form von Oxiden, Mischoxiden und/oder Heteropolyverbindungen und optional Eisen und/oder Molybdän in Form von Halogeniden keine anderen aktive(n) Katalysator-komponente(n).

Der Katalysator (K-1) kann als Vollkatalysator oder als Trägerkatalysator eingesetzt werden.

Geeignete Trägermaterialien sind dem Fachmann bekannt und werden nach Bedarf ausgewählt. Der Träger kann beispielsweise ausgewählt werden aus der Gruppe bestehend aus Magnesiumsilikat, Quarz, Porzellan, Magnesiumoxid, Zinndioxid, Siliziumcarbid, Rutil, Tonerde, Zirkoniumsilikat, Aluminiumsilikat, Cersilikat oder deren Mischungen. Vorzugsweise werden als inerte Trägerstrukturen dem Fachmann an sich bekannte Hohlzylinder oder Ringe eingesetzt. Derartige Trägerstrukturen sind beispielsweise in der WO 2007/059974, der EP 1 127 618 A1 oder der WO 2005/037427 A1 beschrieben. In einer bevorzugten Ausführungsform ist der Katalysator (K-1) jedoch ein Vollkatalysator.

Der Katalysator (K-1) kann durch Kontaktierung von Eisensalzen, Molybdänsalzen, Eisenoxiden, Molybdänoxiden und/oder Eisen-Molybdän-Mischoxiden hergestellt werden.

Beispielsweise kann der Katalysator (K-1) durch Trockenimprägnierung von Eisen(III)nitrat und Ammoniumheptamolybdat hergestellt werden. Ein entsprechendes Verfahren ist beispielsweise in der WO 2010/034880 A2 auf Seiten 18 bis 22 beschrieben.

Außerdem kann der Katalysator (K-1) durch Kontaktierung von Eisen(I)nitrat-nonahydrat ($Fe(NO_3)\cdot 9H_2O$) und Ammoniumheptamolybdathydrat-heptahydrat $(NH_4)_4Mo_7O_{24}\cdot 7H_2O$ hergestellt werden, beziehungsweise durch Kontaktierung von Eisen(III)chlorid ($FeCl_3\cdot 6\ H_2O$) und Ammoniumheptamolybdat-heptahydrat ($(NH_4)_4Mo_7O_{24}\cdot 7H_2O$). Entsprechende Verfahren sind beispielsweise in M. Beale, S. D. Jacques, E. Sacaliuc-Parvalescu, M. G. O'Brien, P. Barnes and B. M. Weckhuysen, Applied Catalysis A: General, 2009, 363(1-2), 143 und J. Gornay, X. Sécordel, G. Tesquet, B. de Ménorval, S. Cristol, P. Fongarland, M. Capron, L. Duhamel, E. Payen, J.-L. Dubois and F. Dumeignil, Green Chem., 2010, 12(10), 1722 beschrieben.

Es können bei der Umsetzung von Methanol (ME) zu Dimethoxymethan (OME 1) und Formaldehyd (FA) sowohl Gasphasenreaktionen als auch Flüssigphasenreaktionen zum Einsatz kommen.

Gasphasenreaktionen werden typischerweise in Festbettreaktoren oder Wirbelschichtreaktoren durchgeführt, während Flüssigphasenreaktionen typischerweise in Festbettreaktoren oder Rührkesselreaktoren erfolgen. Geeignete Reaktoren für Umsetzung von Methanol (ME) zu Formaldehyd (FA) und Dimethoxymethan (OME 1) sind dem Fachmann bekannt und werden nach Bedarf ausgewählt.

Vorzugsweise erfolgt die Umsetzung von Methanol (ME) zu Formaldehyd (FA) und Dimethoxymethan (OME 1) in dem ersten Reaktor (R-1) als Gasphasenreaktion und kann beispielsweise in einem Festbettreaktor durchgeführt werden. Die Reaktionsführung kann im Chargenbetrieb oder im Dauerbetrieb erfolgen, wobei bei einer großtechnischen Anwendung die Reaktoren typischerweise im Dauerbetrieb gefahren werden.

In einer Ausführungsform wird der Reaktor (R-1) bei der Umsetzung von Methanol (ME) zu Dimethoxymethan (OME 1) und Formaldehyd (FA) im Dauerbetrieb gefahren.

In einer Ausführungsform ist die in dem ersten Reaktor (R-1) in Gegenwart des Katalysators (K-1) durchgeführte Umsetzung von Methanol (ME) zu Dimethoxymethan (OME 1) und Formaldehyd (FA) eine Gasphasenreaktion.

In einer bevorzugten Ausführungsform ist die in dem ersten Reaktor (R-1) in Gegenwart des Katalysators (K-1) durchgeführte Umsetzung von Methanol (ME) zu Dimethoxymethan (OME 1) und Formaldehyd (FA) eine Gasphasenreaktion die in einem Festbettreaktor erfolgt.

In einer besonders bevorzugten Ausführungsform ist die in dem ersten Reaktor (R-1) in Gegenwart des Katalysators (K-1) durchgeführte Umsetzung von Methanol (ME) zu Dimethoxymethan (OME 1) und Formaldehyd (FA) eine Gasphasenreaktion die in einem Festbettreaktor erfolgt, welcher im Dauerbetrieb gefahren wird.

Ein wesentlicher Aspekt der vorliegenden Erfindung ist, dass das Methanol (ME), welches vorzugsweise als einziges organisches Edukt dem System von außerhalb zugeführt wird, möglichst effizient zu Polyoxymethylendimethylether (OME), insbesondere zu Polyoxymethylendimethylether mit drei bis fünf repetitiven Einheiten (OME 3-5), umgesetzt wird.

Die Reaktionsbedingungen in dem ersten Reaktor (R-1) sind hierbei von Bedeutung, da sie einen entscheidenden Einfluss auf die Zusammensetzung des aus dem ersten Reaktor (R1) erhaltenen Reaktionsgemisches (RG-1) haben.

Die Reaktion von Methanol (ME) in Gegenwart des Katalysators (K-1) kann bei einer Temperatur in einem Bereich von 50 bis 400 °C durchgeführt werden, vorzugsweise in einem Bereich von 100 bis 350 °C, weiter bevorzugt in einem Bereich von 150 bis 300 °C, noch weiter bevorzugt in einem Bereich von 250 bis 300 °C.

Die Reaktion von Methanol (ME) in Gegenwart des Katalysators (K-1) kann bei einem Druck in einem Bereich von 0,1 bis 5 bar durchgeführt werden, vorzugsweise in einem Bereich von 0,5 bis 3 bar, weiter bevorzugt in einem Bereich von 0,8 bis 1,3 bar, insbesondere bei 1 bar.

Die Reaktion von Methanol (ME) in Gegenwart des Katalysators (K-1) kann bei einer Raumgeschwindigkeit (GHSV) in einem Bereich von 3 000 bis 35 000 $h^{-1}$ durchgeführt werden, vorzugsweise in einem Bereich von 5 000 bis 30 000 $h^{-1}$, weiter bevorzugt in einem Bereich von 6 000 bis 24 000 $h^{-1}$.

In einer bevorzugten Ausführungsform wird die Reaktion von Methanol (ME) in Gegenwart des Katalysators (K-1) bei einer Temperatur in einem Bereich von 250 bis 300 °C, bei einem Druck in einem Bereich von 0,8 bis 1,3 bar und bei einer Raumgeschwindigkeit (GHSV) in einem Bereich von 6 000 bis 24 000 $h^{-1}$ durchgeführt.

Die Effizienz des Verfahrens hängt unter anderem von der Umsatzrate des Methanols (ME) ab. Es ist vorteilhaft wenn eine möglichst große Menge des Methanols (ME), das über den Feed (FD) in den ersten Reaktor (R-1) eingetragen wird, auch umgesetzt wird.

Die Umsatzrate von Methanol ($UR_{ME}$) in dem ersten Reaktor (R-1) kann mindestens 30 mol% betragen, vorzugsweise mindestens 40 mol%, weiter bevorzugt mindestens 50 mol%, bezogen auf die gesamte Stoffmenge des Methanols (ME) dass über den Feed (FD) in den Reaktor (R-1) eingebracht wurde.

Die Umsatzrate ($UR_{ME}$) wird gemäß Formel (III) bestimmt:

$$UR_{ME} = 1 - \frac{NMeOH\ (Rückstand)}{NMeOH\ (Eintrag)}$$

| | |
|---|---|
| $UR_{ME}$ | Umsatzrate des gesamten in einem Umlauf über den Feed (FD) in den Reaktor (R-1) eingebrachten Methanols (ME); |
| $N_{MeOH}$ (Rückstand) | Stoffmenge an Methanol (ME) in dem Reaktionsgemisch (RG-1) unmittelbar vor der Aufarbeitung, durch die das Reaktionsgemisch (RG-1A) oder das Reaktionsgemisch (RG-1B) erhalten wird; |
| $N_{MeOH}$ (Eintrag) | Stoffmenge an Methanol (ME), das in einem Umlauf über den Feed (FD) in den Reaktor (R-1) eingebracht wurde. |

Die Umsatzrate des gesamten "in einem Umlauf' über den Feed (FD) in den Reaktor (R-1) eingebrachten Methanols (ME), beziehungsweise die Stoffmenge an Methanol, (ME) das "in einem Umlauf' über den Feed (FD) in den Reaktor (R-1) eingebracht wurde, berücksichtigt kein Methanol, dass aus dem Reaktionsgemisch (RG-1) oder aus daraus nachfolgend gebildeten Reaktionsgemischen isoliert und in den Reaktor (R-1) recycelt wird.

Es ist insbesondere vorteilhaft, wenn eine möglichst große Menge des Methanols (ME), das über den Feed (FD) in den ersten Reaktor (R-1) eingetragen wird, zu Dimethoxymethan (OME 1) und Formaldehyd (FA) umgesetzt wird.

Die Umsatzrate von Methanol (ME) zu Dimethoxymethan (OME 1) und Formaldehyd (FA) ($UR_{OME1FA}$) in dem ersten Reaktor (R-1), also die Selektivität der Reaktion von Methanol (ME) bezogen auf die Reaktionsprodukte Dimethoxymethan (OME 1) und Formaldehyd (FA), kann mindestens 50 mol% betragen, vorzugsweise mindestens 70 mol%, weiter bevorzugt mindestens 85 mol%, bezogen auf die gesamte Stoffmenge des über den Feed (FD) in den Reaktor (R-1) eingebrachten Methanols (ME).

Die Umsatzrate ($UR_{OME1FA}$) wird gemäß Formel (IV) bestimmt:

$$UR_{OME1FA} = \frac{(N\ (OME1)\ x\ \beta(OME1) + N\ (FA)\ x\ \beta(FA)}{NMeOH\ (Eintrag) - NMeOH\ (Rückstand)} \qquad (IV)$$

| | |
|---|---|
| $UR_{OME1FA}$ | Umsatzrate des gesamten über den Feed (FD) in den Reaktor (R-1) eingebrachten Methanols (ME) zu Dimethoxymethan (OME 1) und Formaldehyd (FA); |
| N (OME1) | Stoffmenge an Dimethoxymethan (OME 1) in dem Reaktionsgemisch (RG-1) unmittelbar vor der Aufarbeitung, durch die das Reaktionsgemisch (RG-1A) oder das Reaktionsgemisch (RG-1B) erhalten wird; |
| N (FA) | Stoffmenge an Formaldehyd (FA) in dem Reaktionsgemisch (RG-1) unmittelbar vor der Aufarbeitung, durch die das Reaktionsgemisch (RG-1A) oder das Reaktionsgemisch (RG-1B) erhalten wird; |
| β (OME1) | Stöchiometrie der Reaktion von Methanol (ME) zu Dimethoxymethan (OME 1), i.e. 3; |
| β (FA) | Stöchiometrie der Reaktion von Methanol (ME) zu Formaldehyd (FA), i.e. 1; |
| $N_{MeOH}$ (Rückstand) | Stoffmenge an Methanol (ME) in dem Reaktionsgemisch (RG-1) unmittelbar vor der Aufarbeitung, durch die das Reaktionsgemisch (RG-1A) oder das Reaktionsgemisch (RG-1B) erhalten wird; |
| $N_{MeOH}$ (Eintrag) | Stoffmenge an Methanol (ME) das in einem Umlauf über den Feed (FD) in den Reaktor (R-1) eingebracht wurde. |

Die Menge der in dem ersten Reaktor (R-1) gebildeten organischen Nebenprodukte, i.e. die Menge an organischen Produkten die in dem ersten Reaktor (R-1) neben Dimethoxymethan (OME 1) und Formaldehyd (FA) gebildet werden, beispielsweise Kohlenstoffmonoxid, Kohlenstoffdioxid, Ameisensäure, Ameisensäuremethylester, und Dimethylether (OME), beträgt höchstens 20 mol%, vorzugsweise höchstens 15 mol%, weiter bevorzugt höchstens 5 mol%, noch weiter bevorzugt höchstens 2 mol%, bezogen auf die gesamte Stoffmenge an Methanol (ME) das über den Feed (FD) in den Reaktor (R-1) eingebracht wurde.

In anderen Worten, die Umsatzrate von Methanol (ME) zu organischen Nebenprodukten ($UR_{NP}$), i.e. organische Produkte die in dem Reaktor (R-1) neben Dimethoxymethan (OME 1) und Formaldehyd (FA) gebildet werden, beträgt vorzugsweise höchstens 20 mol%, weiter vorzugsweise höchstens 15 mol%, noch weiter bevorzugt höchstens 5 mol%, noch weiter bevorzugt höchstens 2 mol%, bezogen auf die gesamte Stoffmenge an Methanol (ME) das über den Feed (FD) in den Reaktor (R-1) eingebracht wurde.

Die Umsatzrate ($UR_{NP}$) wird gemäß Formel (V) bestimmt:

$$UR_{NP} = \frac{\sum N(i) \times \beta(ij)}{NMeOH(Eintrag) - NMeOH(Rückstand)} \quad (V)$$

| | |
|---|---|
| N(i) | Stoffmenge der in dem Reaktor (R-1) gebildeten organischen Nebenprodukte; |
| β(ij) | Stöchiometrie der Reaktion von Methanol (ME) zu dem gebildeten organischen Nebenprodukt i in der Reaktion j |
| $N_{MeOH}$ (Rückstand) | Stoffmenge an Methanol (ME) in dem Reaktionsgemisch (RG-1) unmittelbar vor der Aufarbeitung, durch die das Reaktionsgemisch (RG-1A) oder das Reaktionsgemisch (RG-1B) erhalten wird; |
| $N_{MeOH}$ (Eintrag) | Stoffmenge an Methanol (ME), das in einem Umlauf über den Feed (FD) in den Reaktor (R-1) eingebracht wurde. |

Weiterhin ist es von Vorteil, wenn das Stoffmengenverhältnis von Dimethoxymethan (OME 1) und Formaldehyd (FA) in dem Reaktionsgemisch (RG-1), insbesondere in dem aufgearbeiteten Reaktionsgemisch (RG-1A) oder (RG-1B), in einem bestimmten Bereich liegt, damit eine effiziente Umsetzung zu Polyoxymethylendimethylether (OMEs), insbesondere Polyoxymethylendimethylether mit drei bis fünf repetitiven Einheiten (OME 3-5), möglich ist.

Das Stoffmengenverhältnis (mol/mol) von Dimethoxymethan (OME 1) zu Formaldehyd (FA) in dem Reaktionsgemisch (RG-1) unmittelbar vor der Aufarbeitung, durch die das Reaktionsgemisch (RG-1A) erhalten wird, liegt in einem Bereich von 0,1 bis 2,0, vorzugsweise in einem Bereich von 0,3 bis 1,5, weiter bevorzugt in einem Bereich von 0,3 bis 0,8, noch weiter bevorzugt in einem Bereich von 0,3 bis 0,5, darüber hinaus bevorzugt in einem Bereich von 0,5 bis 1,3 und noch weiter bevorzugt in einem Bereich von 0,8 bis 1,0, ferner bevorzugt in einem Bereich von 0,2 bis 1,5, weiter bevorzugt in einem Bereich von 0,5 bis 1,2.

Das beschriebene Verfahren zeichnet sich unter anderem dadurch aus, dass die aus dem ersten Reaktor (R-1) und dem zweiten Reaktor (R-2) erhaltenen Reaktionsgemische keine aufwändige Aufarbeitung erforderlich machen.

Die in dem zweiten Reaktor (R-2) durchgeführte Umsetzung von Dimethoxymethan (OME 1) und Formaldehyd (FA) zu Polyoxymethylendimethylether (OME), insbesondere Polyoxymethylendimethylether mit drei bis fünf repetitiven Einheiten (OME 3-5), wird durch die Anwesenheit von Methanol (ME) gestört. Das gilt ebenso für das während der Umsetzung von Methanol (ME) zu Dimethoxymethan (OME 1) und Formaldehyd (FA) gebildete Wasser.

Es ist deshalb von Vorteil, in einem Aufarbeitungsschritt verbleibendes Methanol (ME) und Wasser aus dem Reaktionsgemisch (RG-1) zu entfernen.

Das nach dem Aufarbeitungsschritt erhaltene Reaktionsgemisch (RG-1A) oder (RG-1B) enthält immer noch Dimethoxymethan (OME 1) und Formaldehyd (FA), ist aber weitgehend frei von Methanol (ME) und Wasser.

Methanol (ME) und Wasser können beispielsweise über eine fraktionierte Destillation aus dem Reaktionsgemisch (RG-1) entfernt werden. Entsprechende Anlagen sind dem Fachmann bekannt und werden nach Bedarf ausgewählt. Es können jedoch auch andere Verfahren, beispielsweise Membran-, AdsorptionsoderAbsorptionsverfahren, eingesetzt werden, um Methanol (ME) und Wasser aus dem Reaktionsgemisch (RG-1) zu entfernen. Kombinationen der genannten Verfahren sind ebenfalls möglich.

In einer Ausführungsform werden Methanol (ME) und Wasser über eine Destillation, bevorzugt über eine fraktionierte Destillation aus dem Reaktionsgemisch (RG-1) entfernt.

In einer Ausführungsform werden Methanol (ME) und Wasser über einen Adsorptionsprozess und/oder ein Absorptionsverfahren aus dem Reaktionsgemisch (RG-1) entfernt, beispielsweise über die Adsorption und/oder Absorption durch Ionentauscher oder polare Verbindungen, insbesondere über die Adsorption und/oder Absorption durch Zeolithe, Ionentauscherharze oder Polymere mit polaren Gruppen.

In einer Ausführungsform wird Methanol (ME) und Wasser über eine fraktionierte Destillation aus dem Reaktionsgemisch (RG-1) entfernt.

In einer Ausführungsform werden Methanol (ME) und Wasser durch die Behandlung mit einer Membran, vorzugsweise einer Polymermembran mit polaren Gruppen, wie einer Polymermembran umfassend ein Copolymer aus Tetrafluorethylen und Perfluor-3,6-dioxa-4-methyl-7-octen-sulfonsäure aus dem Reaktionsgemisch (RG-1) entfernt. Gemäß einer weiteren Ausführungsform umfasst die Membran Zeolithe.

Es ist selbstverständlich ebenfalls möglich, Methanol (ME) und Wasser über eine fraktionierte Destillation und ein Adsorptionsverfahren und/oder Absorptionsverfahren aus dem Reaktionsgemisch (RG-1) zu entfernen, also ein kombiniertes Verfahren für die Aufarbeitung des Reaktionsgemisches (RG-1) durchzuführen.

Desweiteren ist es ebenfalls möglich, Methanol (ME) und Wasser in einem kombinierten Verfahren über eine Membran und optional ein Adsorptionsverfahren und/oder Absorptionsverfahren aus dem Reaktionsgemisch (RG-1) zu entfernen.

Die in dem ersten Reaktor (R-1) durchgeführte Umsetzung von Methanol (ME) zu Dimethoxymethan (OME 1) und Formaldehyd (FA) erfolgt typischerweise unter einer sauerstoffhaltigen Atmosphäre, beispielsweise unter einer Luftsauerstoffatmosphäre.

Außerdem können während der Reaktion gasförmige Nebenprodukte wie Kohlenstoffmonoxid oder Kohlenstoffdioxid gebildet werden. Es ist vorteilhaft, diese leicht flüchtigen Komponenten aus dem Reaktionsgemisch (RG-1) abzutrennen.

Es kann für die Reaktionsführung von Vorteil sein, die in dem Reaktionsgemisch (RG-1) enthaltenen Bestandteile Methanol (ME), Dimethoxymethan (OME 1), Formaldehyd (FA), Dimethylether (DME) und Wasser zu verflüssigen. Dies ist beispielsweise über eine Absorptions- beziehungsweise Kondensationsanlage möglich. Diese sind dem Fachmann bekannt und werden nach Bedarf ausgewählt.

Wie Eingangs erläutert, ist es eine Aufgabe der vorliegenden Erfindung, ein möglichst effizientes Verfahren zur Herstellung von Polyoxymethylendimethylether (OME), insbesondere ein möglichst effizientes Verfahren zur Herstellung von Polyoxymethylendimethylether mit drei bis fünf repetitiven Einheiten (OME 3-5), bereitzustellen. Dabei ist es von Vorteil wenn möglichst wenige Reaktions- und Aufarbeitungsschritte erforderlich sind. Vorzugsweise werden deshalb neben der Abtrennung von Methanol (ME) und Wasser und einer optional durchgeführten Abtrennung leichtflüchtiger Komponenten wie Sauerstoff, Stickstoff, Kohlenstoffdioxid und/oder Kohlenstoffmonoxid keinerlei weitere Aufarbeitungsschritte an dem Reaktionsgemisch (RG-1), beziehungsweise an dem Reaktionsgemisch (RG-1A) oder (RG-1B), durchgeführt.

In anderen Worten werden aus dem über die Umsetzung in dem ersten Reaktor (R-1) erhaltenen Reaktionsgemisch (RG-1) vorzugsweise neben leicht flüchtigen Komponenten wie Kohlenstoffmonoxid, Kohlenstoffdioxid, Sauerstoff und/oder Stickstoff lediglich nicht umgesetztes Methanol (ME) und Wasser entfernt, bevor das so aufgearbeitete Reaktionsgemisch (RG-1A) oder (RG-1B) in den zweiten Reaktor (R-2) überführt wird.

Das Reaktionsgemisch (RG-1A) und das Reaktionsgemisch (RG-1B) enthalten vorzugsweise neben Dimethoxymethan (OME 1) und Formaldehyd (FA) keinerlei weiterer organischer Bestandteile in einer Stoffmenge über 10 mol%, weiter bevorzugt über 5 mol%, noch weiter bevorzugt über 2 mol%, bezogen auf die gesamte Stoffmenge der organischen Bestandteile des Reaktionsgemisches (RG-1A) bzw. (RG-1B).

Wie oben erläutert, werden für die Herstellung von Polyoxymethylendimethylether (OME), insbesondere für die Herstellung von Polyoxymethylendimethylether mit drei bis fünf repetitiven Einheiten (OME 3-5), gemäß dem erfindungsgemäßen Verfahrens Dimethoxymethan (OME 1) und Formaldehyd (FA) benötigt.

Die Molekulargewichtsverteilung der gebildeten Polyoxymethylendimethylether wird ganz wesentlich von dem Stoffmengenverhältnis zwischen Dimethoxymethan (OME 1) und Formaldehyd (FA) in dem Reaktionsgemisch (RG-1A) bzw. (RG-1B) bestimmt, welches unmittelbar in den zweiten Reaktor (R-2) überführt wird.

Das Stoffmengenverhältnis (mol/mol) von Dimethoxymethan (OME 1) zu Formaldehyd (FA) kann in dem Reaktionsgemisch (RG-1A) bzw. (RG-1B), welches unmittelbar in den zweiten Reaktor (R-2) überführt wird, in einem Bereich von 0,1 bis 2,0 liegen, vorzugsweise in einem Bereich von 0,2 bis 1,5, weiter bevorzugt in einem Bereich von 0,5 bis 1,2 noch weiter bevorzugt in einem Bereich von 0,8 bis 1,0.

Wie oben erläutert, liegt das Stoffmengenverhältnis (mol/mol) von Dimethoxymethan (OME 1) zu Formaldehyd (FA) in dem Reaktionsgemisch (RG-1), welches aus dem ersten Reaktor (R-1) erhalten wurde, ebenfalls in einem Bereich von 0,1 bis 2,0, vorzugsweise in einem Bereich von 0,3 bis 1,5, weiter bevorzugt in einem Bereich von 0,3 bis 0,8, noch weiter bevorzugt in einem Bereich von 0,3 bis 0,5, darüber hinaus bevorzugt in einem Bereich von 0,5 bis 1,3 und noch weiter bevorzugt in einem Bereich von 0,8 bis 1,0, ferner vorzugsweise in einem Bereich von 0,2 bis 1,5, weiter bevorzugt in einem Bereich von 0,5 bis 1,2.

Typischerweise ist das Stoffmengenverhältnis von Dimethoxymethan (OME 1) zu Formaldehyd (FA) in dem Reaktionsgemisch (RG-1) im Vergleich zu dem Stoffmengenverhältnis von Dimethoxymethan (OME 1) zu Formaldehyd (FA) in dem Reaktionsgemisch (RG-1A) bzw. (RG-1B), welches unmittelbar in den zweiten Reaktor (R-2) überführt wird, jedoch zugunsten des Formaldehyds (FA) verschoben. Dies kann beispielsweise durch eine Zugabe von Dimethoxymethan (OME 1) ausgeglichen werden, insbesondere durch die Zugabe von in dem zweiten Reaktor (R-2) nicht umgesetztem Dimethoxymethan (OME 1), welches bei der Aufarbeitung des Reaktionsgemisches (R-2) isoliert wurde.

Das Reaktionsgemisch (RG-1A) bzw. (RG-1B) wird vorzugsweise unmittelbar in den zweiten Reaktor (R-2) überführt. Das Reaktionsgemisch (RG-1A) bzw. (RG-1B), insbesondere das in dem Reaktionsgemisch (RG-1) enthaltene Dimethoxymethan (OME 1) und Formaldehyd (FA), werden anschließend in Gegenwart des zweiten Katalysators (K-2) zu Polyoxymethylendimethylether (OME) umgesetzt, insbesondere zu Polyoxymethylendimethylether mit ein bis 25 repetitiven Einheiten (OME 1-25).

Es ist möglich, das Reaktionsgemisch (RG-1A) bzw. (RG-1B) mit weiteren organischen Komponenten zu versetzen, bevor oder während es in den zweiten Reaktor (R-2) überführt wird, insbesondere mit Dimethoxymethan (OME 1), um das Verhältnis von Dimethoxymethan (OME 1) zu Formaldehyd (FA) einzustellen.

In einer Ausführungsform kann das Reaktionsgemisch (RG-1A) bzw. (RG-1B) mit Polyoxymethylendimethylether mit ein bis zwei repetitiven Einheiten (OME 1-2) versetzt werden, insbesondere mit Polyoxymethylendimethylether mit ein bis zwei repetitiven Einheiten (OME 1-2), welches aus dem Reaktionsgemisch (RG-2) isoliert wurde.

Der Katalysator (K-2) ist vorzugsweise ein Feststoff-Katalysator, insbesondere ein saurer Feststoff-Katalysator. In einer Ausführungsform ist der Katalysator (K-2) ein Feststoff-Katalysator, ausgewählt aus der Gruppe bestehend aus

Ionentauschern, polaren Verbindungen und deren Mischungen, insbesondere aus Zeolithen, Ionentauscherharzen, und Polymeren mit polaren Gruppen, wie Aluminosilicate, Kationentauscherharze, Polymere mit Säuregruppen und deren Mischungen. Entsprechende Katalysatoren sind dem Fachmann bekannt und werden nach Bedarf ausgewählt.

In einer Ausführungsform ist der Katalysator (K-2) ein saures Kationentauscherharz oder ein "Solid Acid" wie Amberlyst®36 von Sigma-Aldrich Chemie GmbH.

Die Umsetzung von Dimethoxymethan (OME 1) und Formaldehyd (FA) in dem zweiten Reaktor (R-2) wird vorzugsweise unter milden Reaktionsbedingungen durchgeführt, um die Bildung von unerwünschten Nebenprodukten zu vermindern.

Die Umsetzung von Dimethoxymethan (OME 1) und Formaldehyd (FA) in dem zweiten Reaktor (R-2) kann bei einer Temperatur in einem Bereich von 20 bis 120 °C durchgeführt werden, vorzugsweise in einem Bereich von 40 bis 100 °C, weiter bevorzugt in einem Bereich von 60 bis 80 °C.

Die Umsetzung von Dimethoxymethan (OME 1) und Formaldehyd (FA) in dem zweiten Reaktor (R-2) kann bei einem Druck in einem Bereich von 0,5 bis 1,5 bar durchgeführt werden, vorzugsweise in einem Bereich von 0,75 bis 1,25 bar, weiter bevorzugt in einem Bereich von 0,95 bis 1,05 bar, insbesondere bei 1 bar.

Die Umsetzung von Dimethoxymethan (OME 1) und Formaldehyd (FA) in dem zweiten Reaktor (R-2) kann bei einer Raumgeschwindigkeit (WHSV) in einem Bereich von 1 bis 15 $h^{-1}$ durchgeführt werden.

In einer bevorzugten Ausführungsform wird die Umsetzung von Dimethoxymethan (OME 1) und Formaldehyd (FA) in dem zweiten Reaktor (R-2) bei einer Temperatur in einem Bereich von 60 bis 80 °C, einem Druck in einem Bereich von 0,95 bis 1,05 bar und einer Raumgeschwindigkeit (WHSV) in einem Bereich von 1 bis 15 $h^{-1}$ durchgeführt.

Das in dem Reaktionsgemisch (RG-1A) bzw. (RG-1B) enthaltene Dimethoxymethan (OME 1) und Formaldehyd (FA) wird in den zweiten Reaktor (R-2) überführt und in Gegenwart des zweiten Katalysators (K-2) zu Polyoxymethylendimethylether (OME) umgesetzt, insbesondere Polyoxymethylendimethylether mit ein bis 25 repetitiven Einheiten (OME 1-25).

Der zweite Reaktor (R-2) kann bei der Umsetzung des Reaktionsgemisches (RG-1A) zu Polyoxymethylendimethylether (OMEs) im Dauerbetrieb gefahren werden.

Die in dem zweiten Reaktor (R-2) in Gegenwart des Katalysators (K-2) durchgeführte Umsetzung des Reaktionsgemisches (RG-1A) bzw. (RG-1B) zu Polyoxymethylendimethylether (OME) kann eine Flüssigphasenreaktion sein.

In einer bevorzugten Ausführungsform ist die in dem zweiten Reaktor (R-2) in Gegenwart des Katalysators (K-2) durchgeführte Umsetzung des Reaktionsgemisches (RG-1A) bzw. (RG-1B) zu Polyoxymethylendimethylether (OME) eine Flüssigphasenreaktion, diese kann beispielsweise in einem Festbettreaktor oder einem Rührkesselreaktor durchgeführt werden.

In einer besonders bevorzugten Ausführungsform ist die in dem zweiten Reaktor (R-2) in Gegenwart des Katalysators (K-2) durchgeführte Umsetzung des Reaktionsgemisches (RG-1A) bzw. (RG-1B) zu Polyoxymethylendimethylether (OME) eine Flüssigphasenreaktion, diese kann beispielsweise in einem Festbettreaktor oder einem Rührkesselreaktor durchgeführt werden, welcher im Dauerbetrieb gefahren wird.

Typischerweise werden nur bestimmte Vertreter der gebildeten Polyoxymethylendimethylether (OME) benötigt, so dass das aus dem zweiten Reaktor erhaltene Reaktionsgemisch (RG-2) aufgearbeitet werden muss.

Die einzelnen bei der Reaktion in dem Reaktor (R-2) gebildeten Polyoxymethylendimethylether (OME) können beispielsweise über eine fraktionierte Destillation voneinander getrennt werden. Entsprechende Anlagen sind dem Fachmann bekannt und werden nach Bedarf ausgewählt.

In einer bevorzugten Ausführungsform werden in einem ersten Aufarbeitungsschritt zunächst niedermolekulare Polyoxymethylendimethylether mit ein bis zwei repetitiven Einheiten (OME 1-2) über eine fraktionierte Destillation aus dem Reaktionsgemisch (RG-2) abgetrennt und anschließend in einem zweiten Aufarbeitungsschritt Polyoxymethylendimethylether mit drei bis fünf repetitiven Einheiten (OME 3-5) über eine weitere fraktionierte Destillation von höhermolekularen Polyoxymethylendimethylethern (OME > 5) abgetrennt.

Das beschriebene Verfahren ist sehr effizient bei der Herstellung von Polyoxymethylendimethylether mit ein bis 25 repetitiven Einheiten (OME 1-25), insbesondere bei der Herstellung von Polyoxymethylendimethylether mit drei bis fünf repetitiven Einheiten (OME 3-5).

Vorzugsweise beträgt das Stoffmengenverhältnis (mol/mol) von Methanol (ME) in dem Feed (FD) zu Polyoxymethylendimethylether mit drei bis fünf repetitiven Einheiten (OME 3-5) in dem aus dem zweiten Reaktor (R-2) erhaltenen Reaktionsgemisch (RG-2) nicht mehr als 1,5, weiter bevorzugt nicht mehr als 1,2, noch weiter bevorzugt nicht mehr als 1,1.

Das beschriebene Verfahren zeichnet sich durch eine hohe Effizienz aus. Die Effizienz des Verfahren (Eff) kann mindestens 40 % betragen, vorzugsweise mindestens 50 %, weiter bevorzugt mindestens 60 %.

Die Effizienz des Verfahren (Eff) wird gemäß Formel (VI) bestimmt, unter der Annahme das 25 % der Produktenergie als Verfahrensenergie verbraucht werden:

$$Eff = \frac{(m(\,OME\ 3{-}6)\ x\ LHV(OME3{-}6))}{(m\ (ME))\ x\ LHV(ME) + E(Verf))} \quad (VI)$$

wobei

| | |
|---|---|
| m (OME 3-6) | Stoffmenge an OME 3-6 in dem aus dem Reaktor (R-2) erhaltenen Reaktionsgemisch (RG-2); |
| m (ME) | Stoffmenge an Methanol (ME) in dem Feed (FD). |
| LHV(OME 3-6) | Unterer Heizwert von OME 3-6 (gemittelt) |
| LHV(ME) | Unterer Heizwert von Methanol (ME) |
| E(Verf) | Verfahrensenergie |

**Figurenbeschreibung:**

Figur 1 zeigt die Reaktionsführung zur Herstellung von Polyoxymethylendimethylether gemäß Beispiel 1.

Figur 2 zeigt die Reaktionsführung zur Herstellung von Polyoxymethylendimethylether gemäß Beispiel 2.

Figur 3 zeigt den Einfluss des Stoffmengenverhältnisses von Dimethoxymethan (OME 1) zu Formaldehyd (FA), variiert über einen Bereich von 1,0 bis 1,8, auf die Selektivität des Katalysators bei der Bildung von Polyoxymethylendimethylether mit ein bis fünf repetitiven Einheiten (OME 1-5).

Figur 4 zeigt den Einfluss des Stoffmengenverhältnisses von Dimethoxymethan (OME 1) zu Formaldehyd (FA), variiert über einen Bereich von 0,33 bis 1, auf die Selektivität des Katalysators bei der Bildung von Polyoxymethylendimethylether mit drei bis fünf repetitiven Einheiten (OME 3-5).

Figur 5 zeigt den Einfluss des aus dem Reaktionsgemisch (RG-2) isolierten Dimethoxymethans (OME 1), welches in das Reaktionsgemisch (RG-1A) zurückgeführt wird, auf die Verteilung der gebildeten Polyoxymethylendimethylether mit drei bis fünf repetitiven Einheiten (OME 3-5).

Figuren 6 bis 8 zeigen den Reaktionsverlauf in dem Reaktor (R-1), bezüglich der Umsetzung von Methanol (ME) zu Dimethoxymethan (OME 1) und Formaldehyd (FA).

**Beispiele:**

Im Folgenden wird die Erfindung anhand von zwei Ausführungsbeispielen näher erläutert.

Beispiel 1:

Der eingesetzte FeMo-Katalysator wird wie folgt hergestellt:
48.5g of $(NH_4)_6Mo_7O_{24}\cdot 4H_2O$ wurden unter Rühren bei 50 °C in 1L destilliertem Wasser gelöst. Anschließend wurden 10 bis 30 mL einer wässrigen HCl-Lösung (39 proz.) zugegeben und ein pH-Wert von 1 eingestellt. 30 g $FeCl_3 \cdot 6\ H_2O$ wurden unter Rühren bei 50 °C in 300 mL destilliertem Wasser gelöst. Die Eisenchlorid-Lösung wurde anschließend innerhalb von 90 s zu der Molybdän-Lösung getropft und bei 50 °C 1h gerührt. Anschließend wurde die Reaktionslösung 8 h gealtert und der gebildete Feststoff durch dekantieren isoliert. Der auf diese Weise erhaltene Pro-Katalysator wurde mit wiederholt mit jeweils 460 mL Wasser gewaschen. Der Pro-Katalysator wurde anschließend unter Vakuum (400 mbar) filtriert und unter Vakuum (900 bis 1000 mbar) bei einer Temperatur von 110 °C für 4 h getrocknet. Der so erhaltene Feststoff wurde mit einer Geschwindigkeit von 2 °C/min (Geschwindigkeitsrampe 2 °C/min) von Raumtemperatur auf eine Temperatur von 550 °C erhitzt und anschließend für 2 h bei 550 °C kalziniert. Der so erhaltene Katalysator wurde gemahlen so das Partikel mit einem Durchmesser von 1-3 mm erhalten wurden.
Das Verfahren zur Herstellung von Polyoxymethylendimethylether mit drei bis sechs repetitiven Einheiten gemäß Beispiel 1 ist in Figur 1 dargestellt. Ein Feed (1) mit einem Gasgemisch aus Stickstoff, Methanol (ME) und Sauerstoff (Stoffmengenverhältnis von Stickstoff zu Methanol (ME) zu Sauerstoff 47:40:13) wurde in den Festbettreaktor R-1 (2) eingeleitet und in Gegenwart des FeMo-Katalysators bei einer Temperatur von 270 °C, einem Druck von 1 bar und einer Raumgeschwindigkeit (GHSV) von 10000 $h^{-1}$ über einen Zeitraum von 8 h umgesetzt. Das so erhaltene Reaktionsgemisch wurde in einem Rückflusskühler (3) eingeleitet und die gebildeten organischen Verbindungen wie

Dimethoxymethan (OME 1) und Formaldehyd (FA), sowie nicht umgesetztes Methanol (ME) und Wasser verflüssigt, durch Kontaktierung mit Polyoxymethylendimethylether mit ein bis zwei repetitiven Einheiten (OME 1-2), welches aus dem Reaktionsgemisch (RG-2) isoliert und in den Rückflusskühler (3) recycelt wurde. Die flüssige Phase wurde über eine erste fraktionierte Destillation (4) aufgearbeitet und das nicht umgesetzte Methanol (ME) und Wasser aus dem Reaktionsgemisch entfernt. Das so aufgearbeitete Reaktionsgemisch wurde in den Festbettreaktor R-2 (5) eingeleitet und in Gegenwart von Amberlyst®A36 der Sigma-Aldrich Chemie GmbH bei einer Temperatur von 60 °C, einem Druck von 1 bar und einer Raumgeschwindigkeit von 3 $h^{-1}$ (WHSV) über einen Zeitraum von 8 h umgesetzt. Das so erhaltene Reaktionsgemisch wurde über eine zweite fraktionierte Destillation (6) aufgearbeitet und Polyoxymethylendimethylether mit ein bis zwei repetitiven Einheiten (OME 1-2) aus dem Reaktionsgemisch entfernt. Das so aufgearbeitete Reaktionsgemisch wurde anschließend über eine dritte fraktionierte Destillation (7) aufgearbeitet und Polyoxymethylendimethylether mit drei bis fünf repetitiven Einheiten (OME 3-5) aus dem Reaktionsgemisch isoliert.

Das nicht umgesetzte Methanol (ME), dass in der ersten fraktionierten Destillation in Form eines Gemisches aus Methanol (ME) und Wasser abgetrennt wurde kann über eine weitere fraktionierte Destillation in einer Säule (8) isoliert und in dem Feed (1) recycelt werden.

Das nicht umgesetzte Dimethoxymethan (OME 1), dass in der zweiten fraktionierten Destillation in Form eines Gemisches aus Dimethoxymethan (OME 1) und Polyoxymethylendimethylether mit zwei repetitiven Einheiten (OME 2) abgetrennt wurde kann über eine Flashsäule (9) isoliert und über den Rückflusskühler (3) recycelt werden.

<u>Beispiel 2:</u>

Der in Beispiel 1 eingesetzte FeMo-Katalysator wird auch in Beispiel 2 eingesetzt.

Das Verfahren zur Herstellung von Polyoxymethylendimethylether mit drei bis fünf repetitiven Einheiten (OME 3-5) gemäß Beispiel 2 ist in Figur 2 dargestellt. Ein Feed (1) mit einem Gasgemisch aus Stickstoff, Methanol (ME) und Sauerstoff (Stoffmengenverhältnis von Stickstoff zu Methanol (ME) zu Sauerstoff 35:56:9) wurde in den Festbettreaktor R-1 (2) eingeleitet und in Gegenwart des FeMo-Katalysators bei einer Temperatur von 260 °C, einem Druck von 1 bar und einer Raumgeschwindigkeit (GHSV) von 13500 $h^{-1}$ über einen Zeitraum von 7,25 h umgesetzt. Das so erhaltene Reaktionsgemisch wurde durch die Polymemembran Perma Pure MD110-144S (3) von Perma Pure LLC geleitet. Auf diese Weise wurden die gebildeten organischen Verbindungen wie Dimethoxymethan (OME 1) und Formaldehyd (FA), von nicht umgesetztem Methanol (ME) und Wasser getrennt. Das so aufgearbeitete Reaktionsgemisch wurde in den Festbettreaktor R-2 (4) eingeleitet und in Gegenwart des Kationentauscherharzes Amberlyst®A36 von Sigma-Aldrich Chemie GmbH bei einer Temperatur von 60 °C, einem Druck von einem bar und einer Raumgeschwindigkeit von 3 $h^{-1}$ (WHSV) über einen Zeitraum von 8 h umgesetzt. Das so erhaltene Reaktionsgemisch wurde über eine erste fraktionierte Destillation (5) aufgearbeitet und Polyoxymethylendimethylether mit ein bis zwei repetitiven Einheiten (OME 1-2) aus dem Reaktionsgemisch entfernt. Das so aufgearbeitete Reaktionsgemisch wurde anschließend über eine zweite fraktionierte Destillation (6) aufgearbeitet und Polyoxymethylendimethylether mit drei bis fünf repetitiven Einheiten (OME 3-5) aus dem Reaktionsgemisch isoliert.

Das nicht umgesetzte Methanol (ME), dass über die Copolymermembran zusammen Wasser aus dem Reaktionsgemisch (RG-1) abgetrennt wurde kann über eine weitere fraktionierte Destillation (7) isoliert und in dem Feed (1) recycelt werden.

**Patentansprüche**

1. Verfahren zur Herstellung von Polyoxymethylendimethylether (OME), umfassend einen ersten Verfahrensschritt, bei dem aus einem Feed (FD), enthaltend Methanol (ME), in einem ersten Reaktor (R-1) in Gegenwart eines ersten Katalysators (K-1) ein Reaktionsgemisch (RG-1) gebildet wird, wobei das Reaktionsgemisch (RG-1) Dimethoxymethan (OME 1) und Formaldehyd (FA) in einem Stoffmengenverhältnis (mol/mol) von Dimethoxymethan (OME 1) zu Formaldehyd (FA) in einem Bereich von 0,1 bis 2,0 umfasst, und einen zweiten Verfahrensschritt, bei dem das Reaktionsgemisch (RG-1) einem zweiten Reaktor (R-2) zugeführt und in Gegenwart eines zweiten Katalysators (K-2) zu Polyoxymethylendimethylether (OME) umgesetzt wird.

2. Verfahren zur Herstellung von Polyoxymethylendimethylether (OME) gemäß Anspruch 1, wobei nicht umgesetztes Methanol (ME) und neben Dimethoxymethan (OME 1) und Formaldehyd (FA) gebildetes Wasser aus dem Reaktionsgemisch (RG-1) entfernt werden.

3. Verfahren zur Herstellung von Polyoxymethylendimethylether (OME), umfassend einen Verfahrensschritt, bei dem aus einem Feed (FD), enthaltend Methanol (ME), in einem Reaktor (R-1) in Gegenwart eines Katalysators (K-1) ein Reaktionsgemisch (RG-1) gebildet wird, wobei das Reaktionsgemisch (RG-1) Dimethoxymethan (OME 1) und

Formaldehyd (FA) umfasst und nicht umgesetztes Methanol (ME) und neben Dimethoxymethan (OME 1) und Formaldehyd (FA) gebildetes Wasser aus dem Reaktionsgemisch (RG-1) entfernt werden.

**4.** Verfahren zur Herstellung von Polyoxymethylendimethylether (OME) gemäß Anspruch 3, umfassend einen zweiten Verfahrensschritt, bei dem das Reaktionsgemisch (RG-1) einem zweiten Reaktor (R-2) zugeführt und in Gegenwart eines zweiten Katalysators (K-2) zu Polyoxymethylendimethylether (OME) umgesetzt wird.

**5.** Verfahren zur Herstellung von Polyoxymethylendimethylether (OME) gemäß einem der Ansprüche 2 bis 4, wobei nicht umgesetztes Methanol (ME) und neben Dimethoxymethan (OME 1) und Formaldehyd (FA) gebildetes Wasser über

i) Destillation und/oder
ii) einen Adsorptionsprozess und/oder
iii) ein Absorptionsverfahren und/oder
iv) eine Membran

aus dem Reaktionsgemisch (RG-1) entfernt werden.

**6.** Verfahren zur Herstellung von Polyoxymethylendimethylether (OME) gemäß einem der vorhergehenden Ansprüche, wobei das Reaktionsgemisch (RG-1) Dimethoxymethan (OME 1) und Formaldehyd (FA) in einem Stoffmengenverhältnis (mol/mol) Dimethoxymethan (OME 1) zu Formaldehyd (FA) in einem Bereich von 0,3 bis 1,5 enthält.

**7.** Verfahren zur Herstellung von Polyoxymethylendimethylether(OME) gemäß einem der vorhergehenden Ansprüche, wobei das Verfahren die folgenden Verfahrensschritte umfasst:

(a) Bereitstellen eines Feed (FD), umfassend Methanol (ME), und überführen in den ersten Reaktor (R-1);
(b) Reaktion von dem in dem Feed (FD) enthaltenem Methanol (ME) in Gegenwart des ersten Katalysators (K-1) unter Bildung eines ersten Reaktionsgemisches (RG-1), wobei das erste Reaktionsgemisch (RG-1) Dimethoxymethan (OME 1) und Formaldehyd (FA) umfasst;
(c) Aufarbeiten des in dem ersten Reaktor (R-1) gebildeten Reaktionsgemisches (RG-1) durch das Abtrennen von Wasser und Methanol über eine Destillation und/oder über einen Adsorptionsprozess und/oder über ein Absorptionsverfahren, so dass ein Reaktionsgemisch (RG-1A) erhalten wird; oder
(d) Aufarbeiten des in dem ersten Reaktor (R-1) gebildeten Reaktionsgemisches (RG-1) durch das Abtrennen von Wasser und Methanol über eine Membran und optional über eine Destillation und/oder über einen Adsorptionsprozess und/oder über ein Absorptionsverfahren, so dass ein Reaktionsgemisch (RG-1B) erhalten wird
(e) Überführen des Reaktionsgemisches (RG-1A) oder des Reaktionsgemisches (RG-1B) in den zweiten Reaktor (R-2);
(f) Reaktion des Reaktionsgemisches (RG-1A) oder des Reaktionsgemisches (RG-1B) in Gegenwart des zweiten Katalysators (K-2) unter Bildung eines Reaktionsgemisches (RG-2), das Reaktionsgemisch (RG-2) umfassend Polyoxymethylendimethylether mit ein bis fünfundzwanzig repetitiven Einheiten (OME 1-25) gemäß Formel (I):

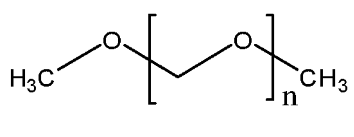 (I)

wobei n = 1-25;
(g) Aufarbeitung des in dem zweiten Reaktor (R-2) gebildeten Reaktionsgemisches (RG-2) durch Abtrennen von Polyoxymethylendimethylether mit drei bis fünf repetitiven Einheiten (OME 3-5) gemäß Formel (II):

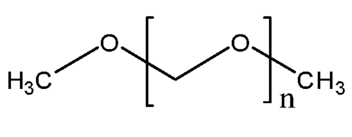 (II)

wobei n = 3-5.

**8.** Verfahren zur Herstellung von Polyoxymethylendimethylether gemäß Anspruch 1 oder 3, wobei der Feed (FD) Methanol (ME) in einer Menge in einem Bereich von 30 bis 90 Vol.-% umfasst, bezogen auf das Gesamtvolumen des Feed (FD).

**9.** Verfahren zur Herstellung von Polyoxymethylendimethylether (OME) gemäß Anspruch 1 oder 3, wobei die Reaktion von Methanol (ME) in Gegenwart des Katalysators (K-1) bei einer Temperatur in einem Bereich von 150 bis 400 °C, bei einem Druck in einem Bereich von 0,5 bis 1,5 bar und einer Raumgeschwindigkeit (GHSV) in einem Bereich von 3000 bis 35000 $h^{-1}$ durchgeführt wird.

**10.** Verfahren zur Herstellung von Polyoxymethylendimethylether gemäß einem der vorhergehenden Ansprüche, wobei der in dem ersten Reaktor (R-1) eingesetzte Katalysator (K-1) Eisen und Molybdän als aktive Katalysatorkomponenten enthält, wobei Eisen und Molybdän vorzugsweise als Oxide, Mischoxide oder deren Mischungen vorliegen, wobei das atomare Stoffmengenverhältnis (mol/mol) von Molybdän zu Eisen in dem Katalysator (K-1) vorzugsweise in einem Bereich von 4,0 bis 2,0 liegt.

**11.** Verfahren zur Herstellung von Polyoxymethylendimethylether (OME) gemäß einem der vorhergehenden Ansprüche, wobei nicht umgesetztes Methanol (ME) und das neben das Dimethoxymethan (OME 1) und Formaldehyd (FA) gebildete Wasser aus dem Reaktionsgemisch (RG-1) über eine Behandlung mit einer Membran entfernt wird.

**12.** Verfahren zur Herstellung von Polyoxymethylendimethylether (OME) gemäß Anspruch 11, wobei es sich bei der Membran um eine Polymermembran mit polaren Gruppen handelt, wobei die Polymermembran vorzugsweise ein Copolymer aus Tetrafluorethylen und Perfluor-3,6-dioxa-4-methyl-7-octen-sulfonsäure umfasst, oder wobei die Membran vorzugsweise Zeolithe umfasst.

**13.** Verfahren zur Herstellung von Polyoxymethylendimethylether (OME) gemäß einem der vorhergehenden Ansprüche, wobei die Stoffmenge der in dem ersten Reaktor (R-1) neben Dimethoxymethan (OME 1) und Formaldehyd (FA) gebildeten organischen Bestandteile weniger als 10 mol% beträgt, bezogen auf die gesamte Stoffmenge der gebildeten organischen Bestandteile in dem Reaktionsgemisch (RG-1).

**14.** Verfahren zur Herstellung von Polyoxymethylendimethylether (OME) gemäß einem der vorhergehenden Ansprüche, wobei wenigstens ein Teil des bei der Aufarbeitung des Reaktionsgemisches (RG-2) isolierten Dimethoxymethans (OME 1) und/oder Polyoxymethylendimethylether mit zwei repetitiven Einheiten (OME 2) mit dem aus dem ersten Reaktor (R-1) erhaltenen Reaktionsgemisch (RG-1) vereint werden.

**15.** Verwendung eines Katalysators (K-1) in einem Verfahren zur Herstellung von Polyoxymethylendimethylether (OME) gemäß einem der vorhergehenden Ansprüche zur Steuerung des aus Methanol (ME) gebildeten Stoffmengenverhältnisses von Dimethoxymethan (OME 1) und Formaldehyd (FA).

## Claims

**1.** Method for producing polyoxymethylene dimethyl ether (OME) comprising a first method step, in which a reaction mixture (RG-1) is formed from a feed (FD) containing methanol (ME) in the presence of a first catalyst (K-l) in a first reactor (R-1), wherein the reaction mixture (RG-1) comprises dimethoxymethane (OME 1) and formaldehyde (FA) in a molar ratio (mol/mol) between dimethoxymethane (OME 1) and formaldehyde (FA) in a range from 0.1 to 2.0, and a second method step, in which the reaction mixture (RG-1) is introduced into a second reactor (R-2) and converted into polyoxymethylene dimethyl ether (OME) in the presence of a second catalyst (K-2).

**2.** Method for producing polyoxymethylene dimethyl ether (OME) according to Claim 1, wherein unreacted methanol (ME) and water formed in addition to dimethoxymethane (OME 1) and formaldehyde (FA) are removed from the reaction mixture (RG-1).

**3.** Method for producing polyoxymethylene dimethyl ether (OME), comprising a method step in which a reaction mixture (RG-1) is formed from a feed (FD) containing methanol (ME) in the presence of a catalyst (K-l) in a reactor (R-1), wherein the reaction mixture (RG-1) comprises dimethoxymethane (OME 1) and formaldehyde (FA), and unreacted methanol (ME) and water formed in addition to dimethoxymethane (OME 1) and formaldehyde (FA) are removed from the reaction mixture (RG-1).

4. Method for producing polyoxymethylene dimethyl ether (OME) according to Claim 3, comprising a second method step, in which the reaction mixture (RG-1) is introduced into a second reactor (R-2) and converted into polyoxymethylene dimethyl ether (OME) in the presence of a second catalyst (K-2).

5. Method for producing polyoxymethylene dimethyl ether (OME) according to any one of Claims 2 to 4, wherein unreacted methanol (ME) and water formed besides dimethoxymethane (OME 1) and formaldehyde (FA) are removed from the reaction mixture (RG-1) by

i) distillation and/or
ii) an adsorption process and/or
iii) an absorption method and/or
iv) a membrane.

6. Method for producing polyoxymethylene dimethyl ether (OME) according to any one of the preceding claims, wherein the reaction mixture (RG-1) contains dimethoxymethane (OME 1) and formaldehyde (FA) in a molar ratio (mol/mol) between dimethoxymethane (OME 1) and formaldehyde (FA) in a range from 0.3 to 1.5.

7. Method for producing polyoxymethylene dimethyl ether (OME) according to any one of the preceding claims, wherein the method comprises the following method steps:

(a) providing a feed (FD) comprising methanol (ME) and transferring it to the first reactor (R-1);
(b) reacting the methanol (ME) contained in the feed (FD) in the presence of the first catalyst (K-1) to form a first reaction mixture (RG-1), wherein the first reaction mixture (RG-1) comprises dimethoxymethane (OME 1) and formaldehyde (FA);
(c) processing the reaction mixture (RG-1) formed in the first reactor (R-1) by separating water and methanol through distillation and/or through an adsorption process and/or through an absorption method, so that a reaction mixture (RG-1A) is obtained; or
(d) processing the reaction mixture (RG-1) formed in the first reactor (R-1) by separating water and methanol through a membrane and optionally through distillation and/or through an adsorption process and/or through an absorption method, so that a reaction mixture (RG-1B) is obtained;
(e) transferring the reaction mixture (RG-1A) or the reaction mixture (RG-1B) to the second reactor (R-2) ;
(f) reacting the reaction mixture (RG-1A) or the reaction mixture (RG-1B) in the presence of the second catalyst (K-2) to form a reaction mixture (RG-2), wherein the reaction mixture (RG-2) comprises polyoxymethylene dimethyl ether with one to twenty-five repetitive units (OME 1-25) according to formula (I):

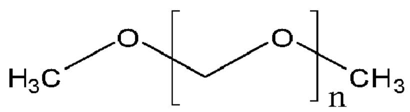 (I)

wherein n = 1-25;
(g) processing the reaction mixture (RG-2) formed in the second reactor (R-2) by separating polyoxymethylene dimethyl ether with three to five repetitive units (OME 3-5) according to formula (II) :

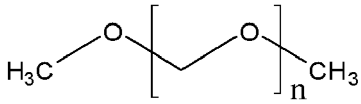 (II)

wherein n = 3-5.

8. Method for producing polyoxymethylene dimethyl ether according to either of Claims 1 or 3, wherein the feed (FD) comprises methanol (ME) in a quantity in a range from 30 to 90% by volume relative to the total volume of the feed (FD).

9. Method for producing polyoxymethylene dimethyl ether (OME) according to either of Claims 1 or 3, wherein the reaction of methanol (ME) is conducted in the presence of the catalyst (K-1) at a temperature in a range from 150 to 400°C, at a pressure in a range from 0.5 to 1.5 bar, and a space velocity (GHSV) in a range from 3000 to 35000 $h^{-1}$.

10. Method for producing polyoxymethylene dimethyl ether according to any one of the preceding claims, wherein the catalyst (K-1) used in the first reactor (R-1) contains iron and molybdenum as active catalyst components, wherein iron and molybdenum are preferably present as oxides, mixed oxides or mixtures thereof, wherein the atomic ratio (mol/mol) between molybdenum and iron in the catalyst (K-1) is preferably in a range from 4.0 to 2.0.

11. Method for producing polyoxymethylene dimethyl ether (OME) according to any one of the preceding claims, wherein unreacted methanol (ME) and the water formed in addition to dimethoxymethane (OME 1) and formaldehyde (FA) is removed from the reaction mixture (RG-1) by a treatment with a membrane.

12. Method for producing polyoxymethylene dimethyl ether (OME) according to Claim 11, wherein the membrane is a polymer membrane with polar groups, wherein the polymer membrane preferably comprises a copolymer of tetrafluoroethylene and perfluoro-3,6-dioxa-4-methyl-7-octene-sulfonic acid, or wherein the membrane preferably comprises zeolites.

13. Method for producing polyoxymethylene dimethyl ether (OME) according to any one of the preceding claims, wherein the amount of substance of the organic constituents formed in the first reactor (R-1) besides dimethoxymethane (OME 1) and formaldehyde (FA) is less than 10 mol% relative to the total amount of substance of the organic constituents in the reaction mixture (RG-1).

14. Method for producing polyoxymethylene dimethyl ether (OME) according to any one of the preceding claims, wherein at least some of the dimethoxymethane (OME 1) and/or polyoxymethylene dimethyl ether with two repetitive unis (OME 2) isolated during the treatment of the reaction mixture (RG-2) is combined with the reaction mixture (RG-1) obtained from the first reactor (R-1).

15. Use of a catalyst (K-1) in a method for producing polyoxymethylene dimethyl ether (OME) according to any one of the preceding claims to control the molar ratio between dimethoxymethane (OME 1) and formaldehyde (FA) formed from methanol (ME).

## Revendications

1. Procédé de fabrication de polyoxyméthylendiméthyléther (OME) comprenant une première étape de procédé, dans laquelle un mélange réactionnel (RG-1) est formé dans un premier réacteur (R-l) en présence d'un premier catalyseur (K-l) à partir d'une alimentation (FD) contenant du méthanol (ME), dans lequel le mélange réactionnel (RG-1) comprend du diméthoxyméthane (OME 1) et du formaldéhyde (FA) dans un rapport molaire (mole/mole) entre le diméthoxyméthane (OME 1) et le formaldéhyde (FA) dans une plage de 0,1 à 2,0, et une seconde étape de procédé, dans laquelle le mélange réactionnel (RG-1) est alimenté dans un second réacteur (R-2) et converti en polyoxyméthylendiméthyléther (OME) en présence d'un second catalyseur (K-2).

2. Procédé de fabrication de polyoxyméthylendiméthyléther (OME) selon la revendication 1, dans lequel le méthanol (ME) non converti et l'eau formée en plus du diméthoxyméthane (OME 1) et du formaldéhyde (FA) sont éliminés du mélange réactionnel (RG-1).

3. Procédé de fabrication de polyoxyméthylendiméthyléther (OME) comprenant une étape de procédé, dans laquelle un mélange réactionnel (RG-1) est formé dans un réacteur (R-l) en présence d'un catalyseur (K-l) à partir d'une alimentation (FD) contenant du méthanol (ME), dans lequel le mélange réactionnel (RG-1) comprend du diméthoxyméthane (OME 1) et du formaldéhyde (FA) et le méthanol (ME) non converti et l'eau formée en plus du diméthoxyméthane (OME 1) et du formaldéhyde (FA) sont éliminés du mélange réactionnel (RG-1).

4. Procédé de fabrication de polyoxyméthylendiméthyléther (OME) selon la revendication 3, comprenant une seconde étape de procédé, dans laquelle le mélange réactionnel (RG-1) est alimenté dans un second réacteur (R-2) et converti en polyoxyméthylendiméthyléther (OME) en présence d'un second catalyseur (K-2).

5. Procédé de fabrication de polyoxyméthylendiméthyléther (OME) selon l'une quelconque des revendications 2 à 4, dans lequel le méthanol (ME) non converti et l'eau formée en plus du diméthoxyméthane (OME 1) et du formaldéhyde (FA) sont éliminés du mélange réactionnel (RG-1) par le biais de

i) la distillation et/ou

ii) un processus d'adsorption et/ou
iii) un procédé d'absorption et/ou
iv) une membrane.

6. Procédé de fabrication de polyoxyméthylendiméthyléther (OME) selon l'une quelconque des revendications précédentes, dans lequel le mélange réactionnel (RG-1) contient du diméthoxyméthane (OME 1) et du formaldéhyde (FA) dans un rapport molaire (mole/mole) entre le diméthoxyméthane (OME 1) et le formaldéhyde (FA) dans une plage de 0,3 à 1,5.

7. Procédé de fabrication de polyoxyméthylendiméthyléther (OME) selon l'une quelconque des revendications précédentes, dans lequel le procédé comprend les étapes suivantes :

(a) la fourniture d'une alimentation (FD) comprenant du méthanol (ME) et le transfert dans le premier réacteur (R-l) ;
(b) la réaction du méthanol (ME) contenu dans l'alimentation (FD) en présence du premier catalyseur (K-l) en formant un premier mélange réactionnel (RG-1), dans lequel le premier mélange réactionnel (RG-1) comprend du diméthoxyméthane (OME 1) et du formaldéhyde (FA) ;
(c) le traitement du mélange réactionnel (RG-1) formé dans le premier réacteur (R-l) par la séparation de l'eau et du méthanol par le biais d'une distillation et/ou d'un processus d'adsorption et/ou d'un procédé d'absorption de manière à obtenir un mélange réactionnel (RG-1A) ; ou
(d) le traitement du mélange réactionnel (RG-1) formé dans le premier réacteur (R-l) par la séparation de l'eau et du méthanol par le biais d'une membrane et, facultativement, par le biais d'une distillation et/ou d'un processus d'adsorption et/ou par le biais d'un procédé d'absorption de manière à obtenir un mélange réactionnel (RG-1B) ;
(e) le transfert du mélange réactionnel (RG-1A) ou du mélange réactionnel (RG-1B) dans le second réacteur (R-2) ;
(f) la réaction du mélange réactionnel (RG-1A) ou du mélange réactionnel (RG-1B) en présence du second catalyseur (K-2) en formant un mélange réactionnel (RG-2), le mélange réactionnel (RG-2) comprenant du polyoxyméthylendiméthyléther avec une à vingt-cinq unités répétitives (OME 1-25) selon la formule (I) :

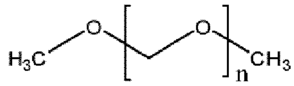 (I)

dans lequel n = 1-25 ;
(g) le traitement du mélange réactionnel (RG-2) formé dans le second réacteur (R-2) par séparation du polyoxyméthylendiméthyléther avec trois à cinq unités répétitives (OME 3-5) selon la formule (II) :

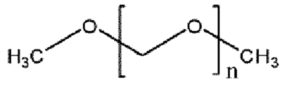 (II)

dans lequel n = 3-5.

8. Procédé de fabrication de polyoxyméthylendiméthyléther selon la revendication 1 ou 3, dans lequel l'alimentation (FD) comprend du méthanol (ME) en une quantité dans une plage de 30 à 90 % en volume, par rapport au volume total de l'alimentation (FD).

9. Procédé de fabrication de polyoxyméthylendiméthyléther (OME) selon la revendication 1 ou 3, dans lequel la réaction du méthanol (ME) est effectuée en présence du catalyseur (K-l) à une température dans une plage de 150 à 400 °C, à une pression dans une plage de 0,5 à 1,5 bar et à une vitesse ambiante (GHSV) dans une plage de 3000 à 35000 $h^{-1.}$

10. Procédé de fabrication de polyoxyméthylendiméthyléther selon l'une quelconque des revendications précédentes, dans lequel le catalyseur (K-l) utilisé dans le premier réacteur (R-l) contient du fer et du molybdène comme composants catalyseurs actifs, dans lequel le fer et le molybdène sont de préférence présents sous forme d'oxydes, d'oxydes mixtes ou de mélanges de ceux-ci, dans lequel le rapport molaire atomique (mole/mole) entre le molybdène et le fer dans le catalyseur (K-l) se situe de préférence dans une plage de 4,0 à 2,0.

**11.** Procédé de fabrication de polyoxyméthylendiméthyléther (OME) selon l'une quelconque des revendications précédentes, dans lequel le méthanol (ME) non converti et l'eau formée en plus du diméthoxyméthane (OME 1) et du formaldéhyde (FA) sont éliminés du mélange réactionnel (RG-1) par le biais d'un traitement avec une membrane.

**12.** Procédé de fabrication de polyoxyméthylendiméthyléther (OME) selon la revendication 11, dans lequel il s'agit pour la membrane d'une membrane polymère à groupes polaires, dans lequel la membrane polymère comprend de préférence un copolymère de tétrafluoroéthylène et d'acide perfluoro-3,6-dioxa-4-méthyl-7-octène-sulfonique, ou dans lequel la membrane comprend de préférence des zéolithes.

**13.** Procédé de fabrication de polyoxyméthylendiméthyléther (OME) selon l'une quelconque des revendications précédentes, dans lequel la quantité de matière des composants organiques formés dans le premier réacteur (R-I) en plus du diméthoxyméthane (OME 1) et du formaldéhyde (FA) est inférieure à 10 % en mole, par rapport à la quantité totale de matière des composants organiques formés dans le mélange réactionnel (RG-1).

**14.** Procédé de fabrication de polyoxyméthylendiméthyléther (OME) selon l'une quelconque des revendications précédentes, dans lequel au moins une partie du diméthoxyméthane (OME 1) et/ou du polyoxyméthylendiméthyléther à deux unités répétitives (OME 2) isolé lors du traitement du mélange réactionnel (RG-2) est associée au mélange réactionnel (RG-1) obtenu à partir du premier réacteur (R-I).

**15.** Utilisation d'un catalyseur (K-I) dans un procédé de fabrication de polyoxyméthylendiméthyléther (OME) selon l'une quelconque des revendications précédentes pour commander le rapport molaire entre le diméthoxyméthane (OME 1) et le formaldéhyde (FA) formé à partir du méthanol (ME).

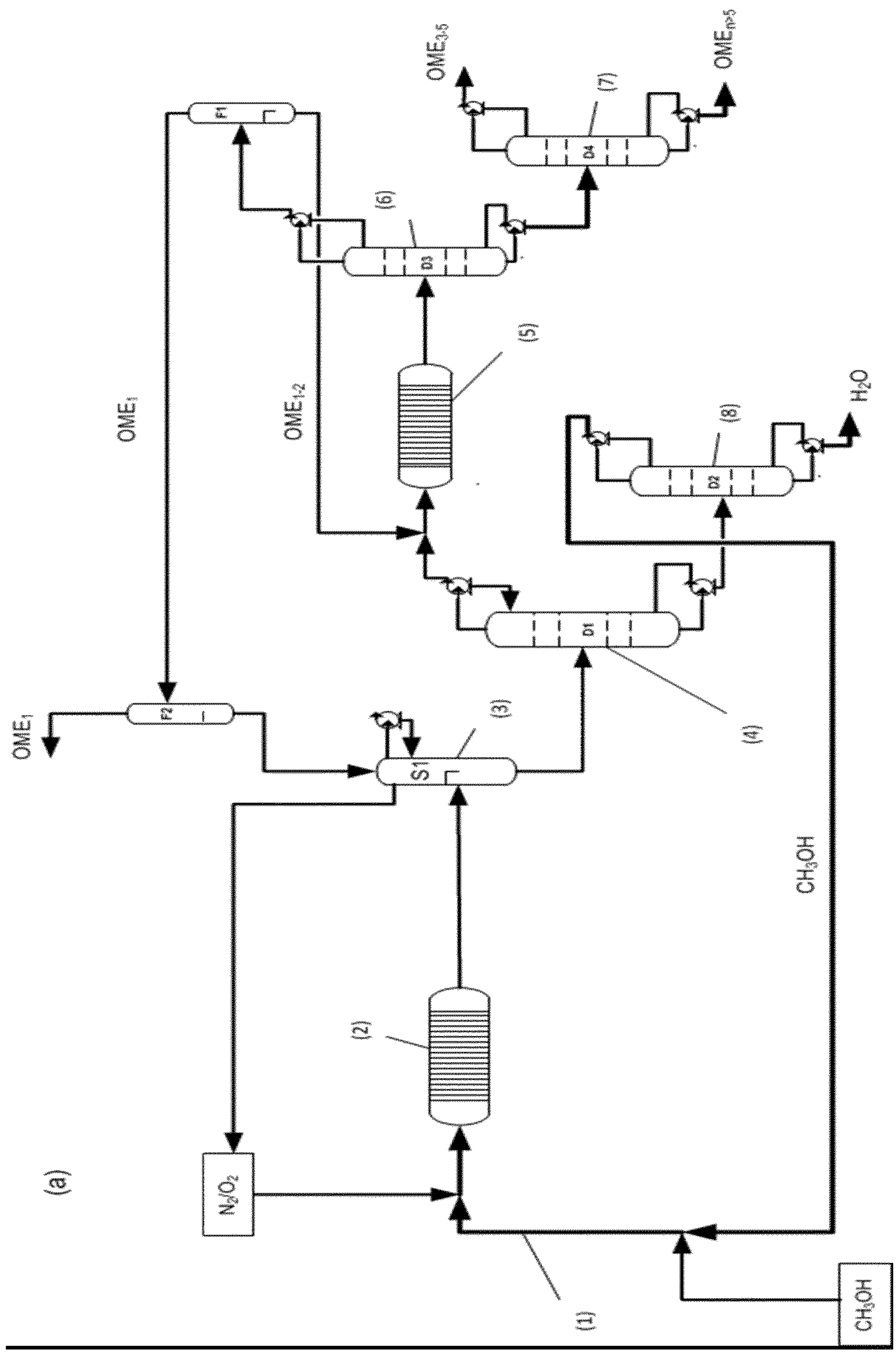
(a)
N2/O2
(1)
(2)
(3)
(4)
(5)
(6)
(7)
(8)
S1
F1
F2
D1
D2
D3
D4
OME1
OME1
OME1-2
OME3-5
OMEn>5
H2O
CH3OH
CH3OH

**Figur 1**

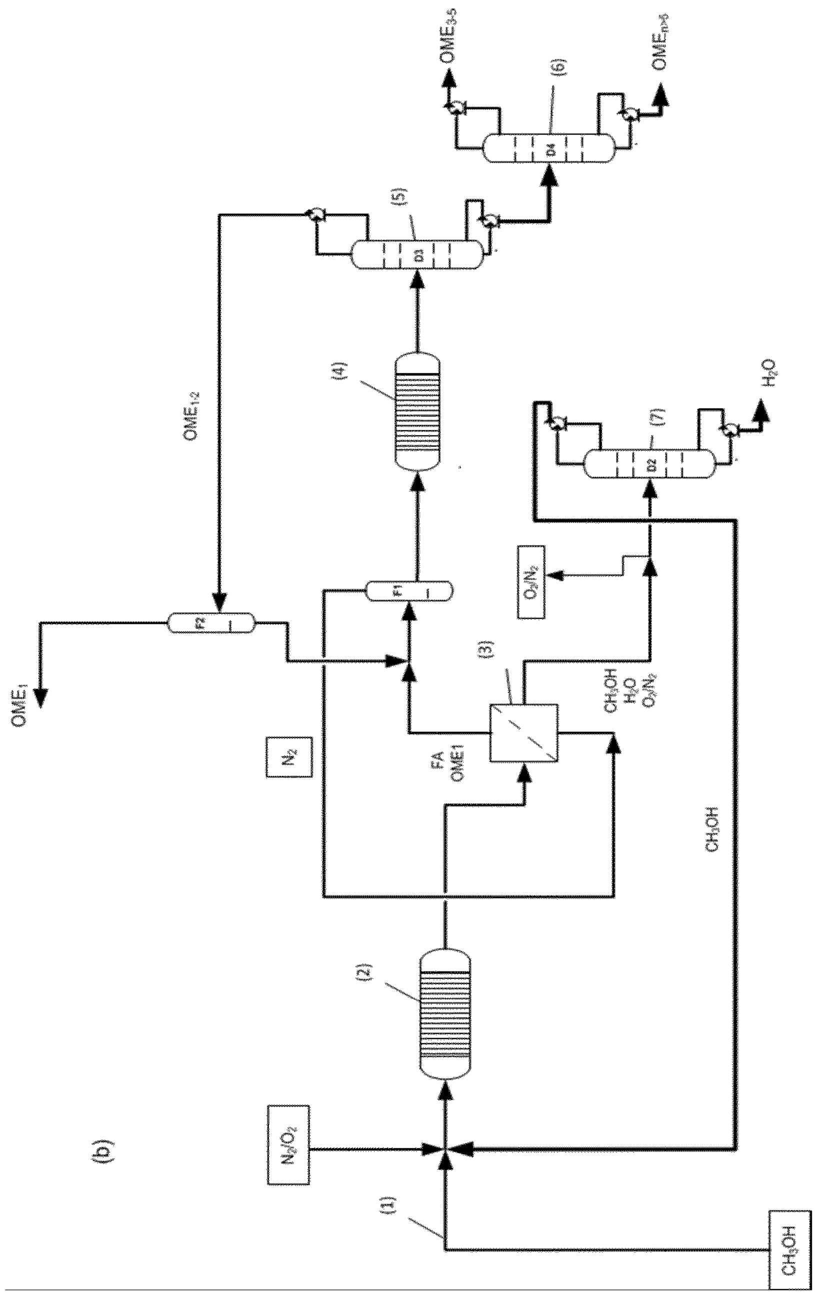
(b)
$CH_3OH$
(1)
$N_2/O_2$
(2)
$N_2$
FA
OME1
(3)
$CH_3OH$
$H_2O$
$O_2/N_2$
$CH_3OH$
$O_2/N_2$
F1
F2
$OME_1$
$OME_{1-2}$
(4)
(5)
D3
(6)
D4
$OME_{3-5}$
$OME_{n>5}$
(7)
D2
$H_2O$

**Figur 2**

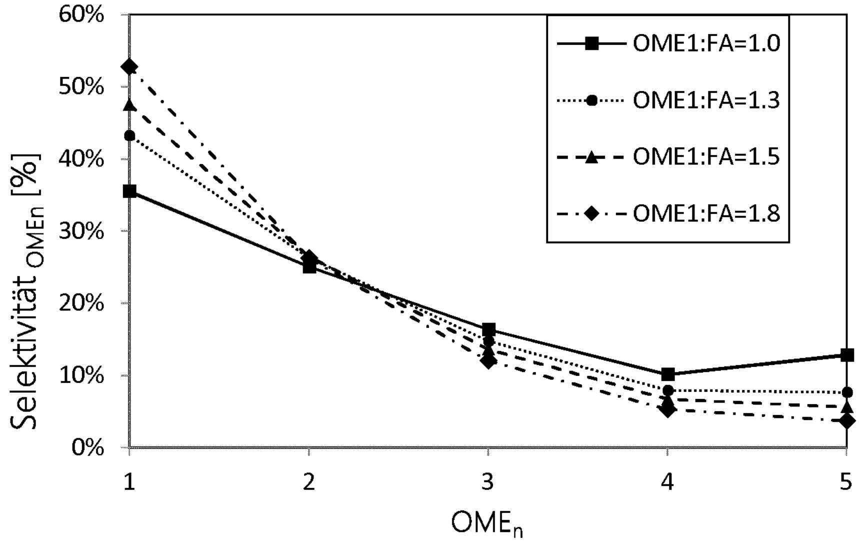


Figur 3

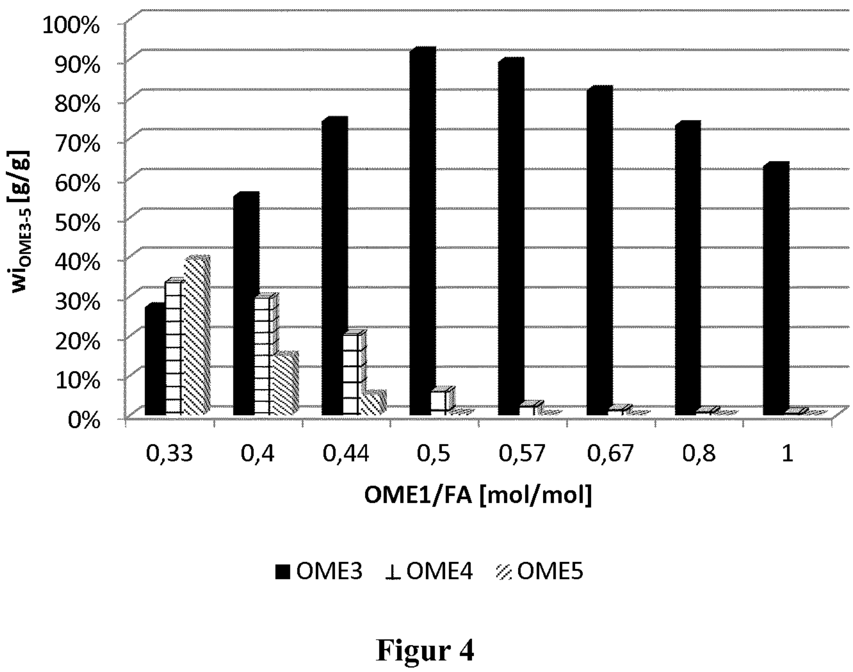


Figur 4

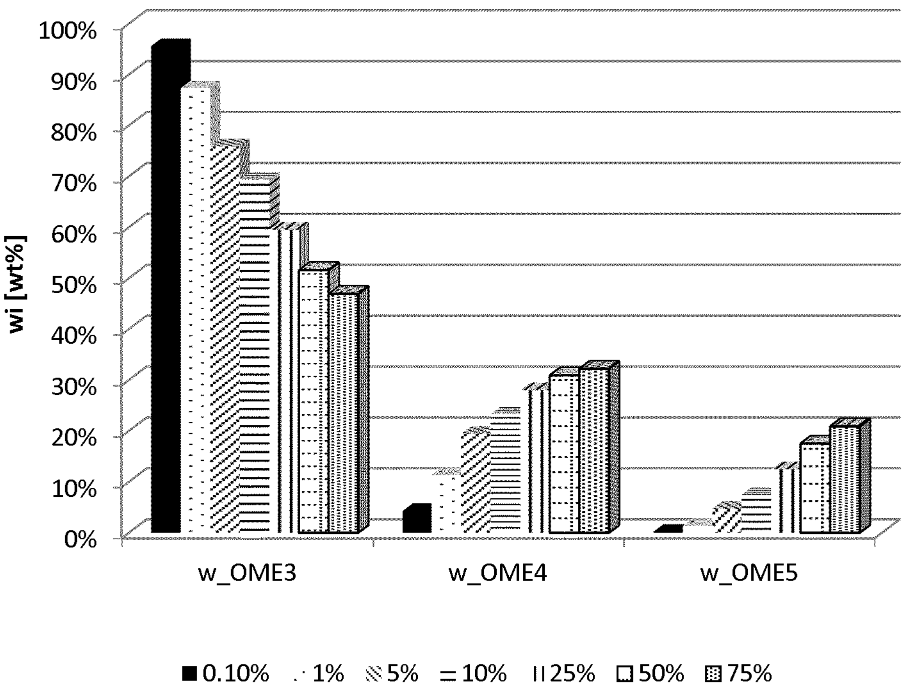
100%
90%
80%
70%
60%
50%
40%
30%
20%
10%
0%
wi [wt%]
w_OME3
w_OME4
w_OME5
0.10%
1%
5%
10%
25%
50%
75%

**Figur 5**

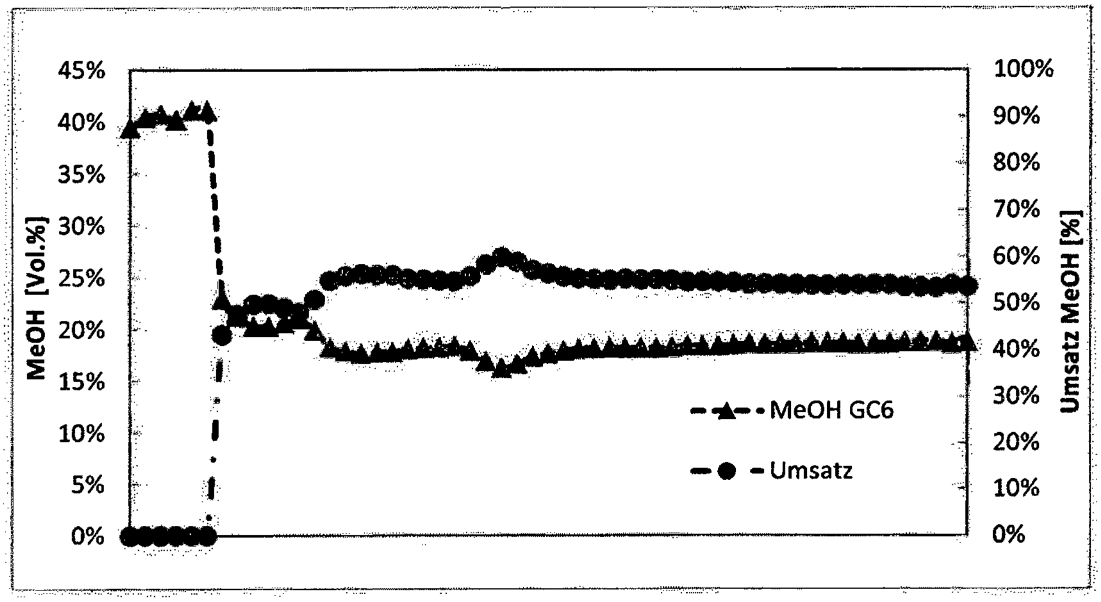


Figur 6

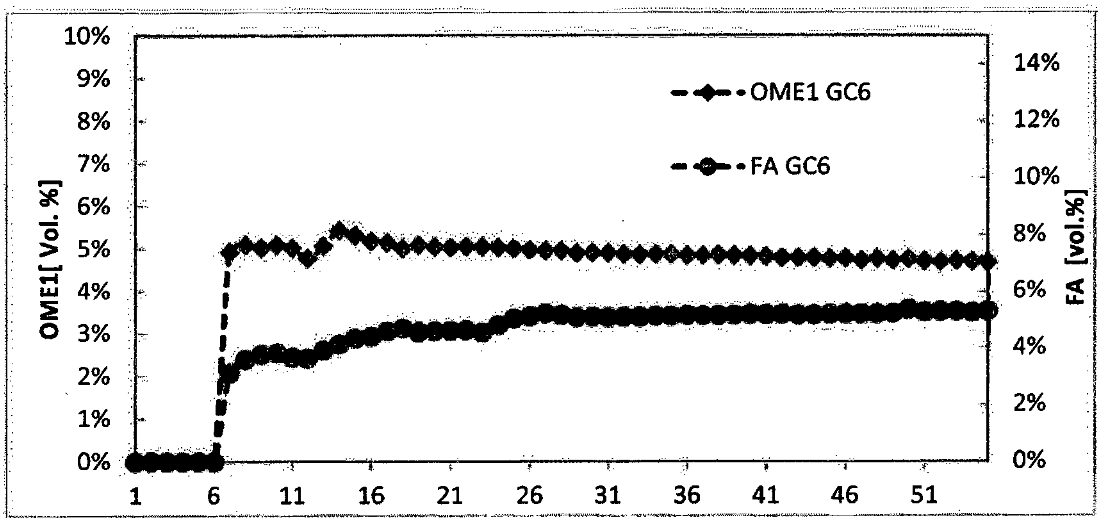


Figur 7

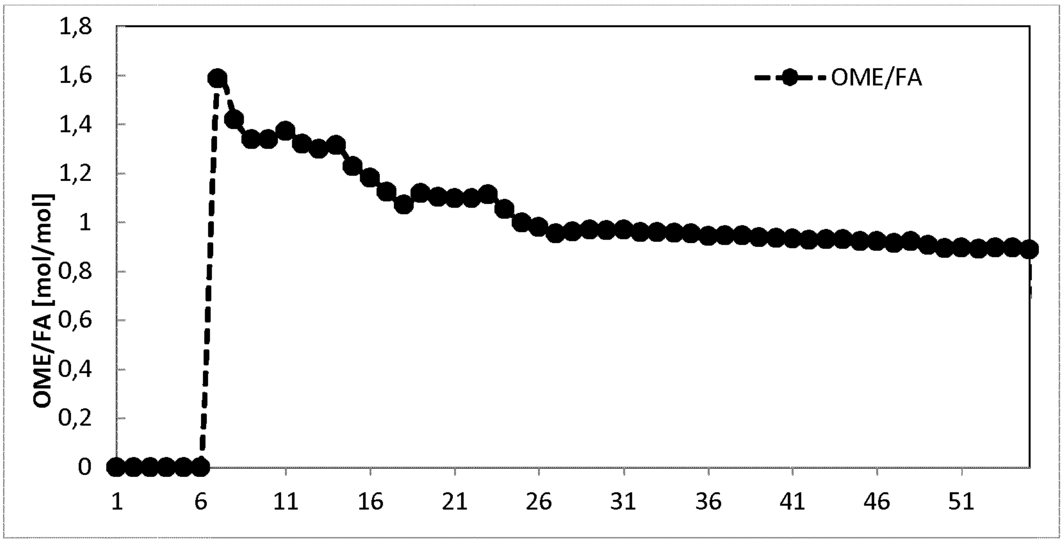
1,8
1,6
1,4
1,2
1
0,8
0,6
0,4
0,2
0
OME/FA [mol/mol]
OME/FA
1
6
11
16
21
26
31
36
41
46
51

Figur 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2228359 A1
- WO 2007059974 A
- EP 1127618 A1
- WO 2005037427 A1
- WO 2010034880 A2


### In der Beschreibung aufgeführte Nicht-Patentliteratur


- **J. GOMAY ; X. SÉCORDEL ; G. TESQUET ; B. DE MÉNORVAL ; S. CRISTOL ; P. FONGARLAND ; M. CAPRON ; L. DUHAMEL ; E. PAYEN ; J.-L. DUBOIS.** *Green Chem,* 2010, vol. 12 (10), 1722
- **J. M. TATIBOUËT.** *Applied Catalysis A: General,* 1997, vol. 148 (2), 213
- **K.-A. THAVORNPRASERT ; M. CAPRON ; L. JALOWIECKI-DUHAMEL ; F. DUMEIGNIL.** *Catal. Sci. Technol.,* 2016, vol. 6 (4), 958
- **M. BEALE ; S. D. JACQUES ; E. SACALIUC-PARVALESCU ; M. G. O'BRIEN ; P. BARNES ; B. M. WECKHUYSEN.** *Applied Catalysis A: General,* 2009, vol. 363 (1-2), 143
- **J. GORNAY ; X. SÉCORDEL ; G. TESQUET ; B. DE MÉNORVAL ; S. CRISTOL ; P. FONGARLAND ; M. CAPRON ; L. DUHAMEL ; E. PAYEN ; J.-L. DUBOIS.** *Green Chem.,* 2010, vol. 12 (10), 1722